(19) **Europäisches Patentamt European Patent Office Office européen des brevets**

(11) **EP 3 574 829 B1**

(12) **EUROPEAN PATENT SPECIFICATION**

(45) Date of publication and mention of the grant of the patent:
**03.05.2023 Bulletin 2023/18**

(21) Application number: **18744637.2**

(22) Date of filing: **22.01.2018**

(51) International Patent Classification (IPC):
**A61B 5/11** *(2006.01)*    **A61B 5/107** *(2006.01)*
**A61B 5/024** *(2006.01)*    **A61B 5/00** *(2006.01)*

(52) Cooperative Patent Classification (CPC):
**A61B 5/1116; A61B 5/024; A61B 5/1071; A61B 5/1118; A61B 5/112; A61B 5/72;** A61B 2562/0219

(86) International application number:
**PCT/JP2018/001740**

(87) International publication number:
**WO 2018/139398 (02.08.2018 Gazette 2018/31)**

(54) **ACTIVITY STATE ANALYSIS DEVICE AND METHOD**

AKTIVITÄTSZUSTANDSANALYSEVORRICHTUNG UND -VERFAHREN

DISPOSITIF ET PROCÉDÉ D'ANALYSE D'ÉTAT D'ACTIVITÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.01.2017   JP 2017013964**

(43) Date of publication of application:
**04.12.2019   Bulletin 2019/49**

(73) Proprietor: **Nippon Telegraph And Telephone Corporation**
**Chiyoda-ku, Tokyo 100-8116 (JP)**

(72) Inventors:
- **OGASAWARA, Takayuki**
  **Musashino-shi**
  **Tokyo 180-8585 (JP)**
- **TSUKADA, Shingo**
  **Musashino-shi**
  **Tokyo 180-8585 (JP)**
- **OSHIMA, Shoichi**
  **Musashino-shi**
  **Tokyo 180-8585 (JP)**
- **MORIMURA, Hiroki**
  **Musashino-shi**
  **Tokyo 180-8585 (JP)**
- **NAKASHIMA, Hiroshi**
  **Musashino-shi**
  **Tokyo 180-8585 (JP)**
- **SATO, Rieko**
  **Musashino-shi**
  **Tokyo 180-8585 (JP)**

(74) Representative: **Samson & Partner Patentanwälte mbB**
**Widenmayerstraße 6**
**80538 München (DE)**

(56) References cited:
| | |
|---|---|
| JP-A- 2004 081 632 | JP-A- 2006 271 893 |
| JP-A- 2010 125 239 | JP-A- 2010 207 488 |
| JP-A- 2017 500 076 | US-A1- 2002 170 193 |
| US-A1- 2012 274 554 | US-A1- 2014 313 030 |
| US-A1- 2015 126 822 | US-A1- 2016 100 776 |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The present invention relates to an activity state analysis device and method and, more particularly, to an activity state analysis device and method for analyzing the activity state of a measurement target person based on physical information measured by a sensor attached to the measurement target person.

Background Art

In recent years, there have been proposed techniques of detecting the physical information of a user (measurement target person) from a sensor attached to the measurement target person. As one of such physical information measurement techniques, for example, non-patent literature 1 proposes a technique of calculating the posture of a measurement target person from acceleration data measured by a three-axis acceleration sensor configured to detect accelerations in three directions along X-, Y-, and Z-axes, grasping a physical activity from the posture, and making use of it for lifestyle investigations (see, for example, p.67 of non-patent literature 1).

In non-patent literature 1, it is estimated based on the average value of the three-axis accelerations whether the posture of the measurement target person is a lying position or a standing position. Furthermore, in non-patent literature 1, four directions (LyingLeft, LyingRight, LyingFaceUp, and LyingFaceDown) are calculated in the lying position, and the tilt angle of the state of the measurement target person is calculated in the standing position from the average value of the axes.

US 2015/126822 A1 relates to congestive heart failure risk status determination methods and related devices and discloses a device and method according to the preamble of the independent claims.

US 2012/274554 A1 relates to a body movement detection device and related display control method.

US 2016/100776 A1 relates to fall detection and fall risk detection systems and methods.

US 2014/313030 A1 relates to bed exit monitoring apparatus.

Related Art Literature

Patent Literature

Patent Literature 1: Japanese Patent Laid-Open No. 2016-182160

Non-Patent Literature

Non-Patent Literature 1: "hitoe transmitter SDK API manual" NTT DOCOMO, Inc., November 14, 2016.
Non-Patent Literature 2: Ken Koda, "Meanings and Importance of Active Bed Leaving and Exercise Load and Its Physiological Mechanism", Human Resources Development Study Group for Community Rehabilitation, 2013, http://wakayama-med-reha.com/wp-content/uploads/2013/12/31bc2df5f92f959aaabc9d676a556abe .pdf.
Non-Patent Literature 3:

https://ja.wikipedia.org/wiki/運動強度

Disclosure of Invention

Problem to be Solved by the Invention

In the above-described technique of posture determination using the acceleration sensor, however, if the measurement target person takes an exceptional position at the time of data acquisition, the determination is erroneously done.

For example, when the measurement target person in the standing position bends forward only for several sec to re-tie the shoelaces, the position is erroneously determined as lying face down (LyingFaceDown) by the above-described technique. Such a determination error may be allowed for an application purpose such as motion capture but is considerably problematic in a case in which the activity state of a body is measured with focus on the continuation time of the state as in lifestyle investigations.

For example, in the field of rehabilitation, it has been pointed out that adverse effects such as hypotension readily occurs if a lying state continues, and the importance of a sitting position/standing position is pointed out (non-patent literature 2). From such a viewpoint, focus is placed on the continuation period of the activity state of a patient. In general, the temporal scale of the continuation period is several hrs to several days or several weeks. In the lifestyle investigations, the above-described determination of lying face down, which is done at the time of re-tying shoelaces, should be excluded as a temporary exception as a disturbance in grasping the continuity because it may lead to misunderstanding rather than giving convenience. As described above, the conventional technique cannot be applied in a case of measuring an activity state as a habit.

The present invention has been made to solve the above-described problem, and has as its object more correctly measure the activity state of a measurement target person with focus on the lifestyle.

Means of Solution to the Problem

According to the present invention, there is provided an activity state analysis device comprising a measurement unit attached to a measurement target person and configured to measure an acceleration, a tilt calculation unit configured to obtain an angle of a tilt of an upper part of a body of the measurement target person based on the acceleration measured by the measurement unit, a posture determination unit configured to determine a posture of the measurement target person based on the angle of the tilt obtained by the tilt calculation unit, a body motion calculation unit configured to obtain a magnitude of a body motion of the measurement target person based on the acceleration measured by the measurement unit, an activity state determination unit configured to determine, based on the posture determined by the posture determination unit and the magnitude of the body motion calculated by the body motion calculation unit, whether an activity state of the measurement target person is a first state or a second state different from the first state, an activity state correction unit configured to, if, in a time series of the activity state obtained by the activity state determination unit, the activity state transitions from one state of the first state and the second state to the other state, and the other state continues for a predetermined time defined in advance, determine that the activity state has transitioned from the one state to the other state, and if the other state does not continue for the predetermined time, determine that the activity state has not transitioned from the one state to the other state, and a time correction unit configured to, when the activity state correction unit determines that the activity state has transitioned from the one state to the other state, set a time set back by the predetermined time from a time at which the activity state correction unit determines that the activity state has transitioned from the one state to the other state, as a transition time at which the activity state has transitioned from the one state to the other state.

In the above-described activity state analysis device, the measurement unit may measure accelerations in three directions along X-, Y-, and Z-axes that are orthogonal to each other.

The above-described activity state analysis device may comprise walking pace calculation unit configured to obtain a walking pace of the measurement target person based on the acceleration measured by the measurement unit, and a walking period specifying unit configured to specify, based on the walking pace of the measurement target person obtained by the walking pace calculation unit, a period of a walking state in which the measurement target person was walking in a period of the first state defined by the transition time corrected by the time correction unit.

The above-described activity state analysis device may further comprise a data adjustment unit configured to downsample time-serially obtained data of the activity state including the first state and the second state, while assigning a priority to each state of the first state and the second state.

The above-described activity state analysis device may further comprise a data additional adjustment unit configured to downsample time-series data of the activity state output from the data adjustment unit, while assigning the priority to each state.

The above-described activity state analysis device may further comprise a physical information measurement unit configured to measure physical information of the measurement target person, and a statistic value calculation unit configured to obtain, based on the activity state defined by the transition time corrected by the time correction unit, a statistic value including at least one of an average value, a median, a maximum value, a minimum value, a standard deviation, a 75% level value, and a 25% level value of the physical information measured by the physical information measurement unit.

The above-described activity state analysis device may further comprise a heart rate measurement unit configured to measure a heart rate of the measurement target person, a motion intensity calculation unit configured to calculate a motion intensity of the measurement target person based on the heart rate calculated by the heart rate measurement unit, and a statistic value calculation unit configured to obtain, based on the activity state defined by the transition time corrected by the time correction unit, a statistic value including at least one of an average value, a median, a maximum value, a minimum value, a standard deviation, a 75% level value, and a 25% level value of the motion intensity obtained by the motion intensity calculation unit.

In the above-described activity state analysis device, the first state is a rising state in which the measurement target person gets up, and the second state is a lying state in which the measurement target person is lying in a bed.

According to the present invention, there is also provided an activity state analysis method comprising a first step of measuring an acceleration in an action of a measurement target person, a second step of obtaining an angle of a tilt of an upper part of a body of the measurement target person based on the acceleration measured in the first step, a third step of determining a posture of the measurement target person based on the angle of the tilt obtained in the second step, a fourth step of obtaining a magnitude of a body motion of the measurement target person based on the acceleration measured in the first step, a fifth step of determining, based on the posture determined in the third step and the magnitude of the body motion calculated in the fourth step, whether an activity state of the measurement target person is a first state or a second state different from the first state, a sixth step of, if, in a time series of the activity state obtained in the fifth step, the activity state transitions from one state of the first state and the second state to the other state, and the other state continues for a predetermined time defined in advance, determining that the activity state has transitioned from the one state to the other state, and if the other state does not continue for the predetermined time, determining that the activity state has not transitioned from the one state to the other state, and a seventh step of, when it is determined in the sixth step that the activity state has transitioned from the one state to the other state, setting a time set back by the predetermined time from a time at which it is determined in the sixth step that the activity state has transitioned from the one state to the other state, as a transition time at which the activity state has transitioned from the one state to the other state.

In the above-described activity state analysis method, in the first step, accelerations in three directions along X-, Y-, and Z-axes that are orthogonal to each other are measured.

The above-described activity state analysis method may further comprise an eighth step of obtaining a walking pace of the measurement target person based on the acceleration measured in the first step, and a ninth step of specifying, based on the walking pace of the measurement target person obtained in the eighth step, a period of a walking state in which the measurement target person was walking in a period of the first state defined by the transition time corrected in the seventh step.

The above-described activity state analysis method may further comprise a 10th step of measuring physical information of the measurement target person, and an 11th step of obtaining, based on the activity state defined by the transition time corrected in the seventh step, a statistic value including at least one of an average value, a median, a maximum value, a minimum value, a standard deviation, a 75% level value, and a 25% level value of the physical information measured in the 10th step.

The above-described activity state analysis method may further comprise a 10th step of measuring a heart rate of the measurement target person, an 11th step of calculating a motion intensity of the measurement target person based on the heart rate calculated in the 10th step, and a 13th step of obtaining, based on the activity state defined by the transition time corrected in the seventh step, a statistic value including at least one of an average value, a median, a maximum value, a minimum value, a standard deviation, a 75% level value, and a 25% level value of the motion intensity obtained in the 11th step.

In the above-described activity state analysis method, the first state is a rising state in which the measurement target person gets up, and the for example, is a lying state in which the measurement target person is lying in a bed.

Effect of the Invention

As described above, according to the present invention, in addition to the magnitude of the body motion obtained by the body motion calculation unit, correction is performed when the state after the transition continues for the predetermined time defined in advance, and a delay caused by the correction is corrected. It is therefore possible to obtain an excellent effect of more correctly measuring the activity state of the measurement target person with focus on the lifestyle.

Brief Description of Drawings

Fig. 1 is a block diagram showing the arrangement of an activity state analysis device according to the first embodiment of the present invention;
Fig. 2A is an explanatory view for explaining an effect obtained by the activity state analysis device according to the first embodiment of the present invention;
Fig. 2B is an explanatory view for explaining an effect obtained by the activity state analysis device according to the first embodiment of the present invention;
Fig. 2C is an explanatory view for explaining an effect obtained by the activity state analysis device according to the first embodiment of the present invention;
Fig. 2D is an explanatory view for explaining an effect obtained by the activity state analysis device according to the first embodiment of the present invention;

Fig. 3 is an explanatory view showing the time-series change of an activity state in a case in which the activity state analysis device according to the first embodiment of the present invention is not used;

Fig. 4 is an explanatory view showing the time-series change of an activity state in a case in which the activity state analysis device according to the first embodiment of the present invention is used;

Fig. 5 is a view for explaining a system using the activity state analysis device according to the first embodiment;

Fig. 6 is a block diagram showing the arrangement of the system using the activity state analysis device according to the first embodiment;

Fig. 7 is a block diagram showing the arrangement of an activity state analysis device according to the second embodiment of the present invention;

Fig. 8 is a characteristic chart showing the time-series change of an activity state obtained by the activity state analysis device according to the second embodiment;

Fig. 9 is a block diagram showing the arrangement of an activity state analysis device according to the third embodiment of the present invention;

Fig. 10 is a characteristic chart showing the time-series change of a statistic value concerning the heart rate of a measurement target person obtained by the activity state analysis device according to the third embodiment;

Fig. 11 is a block diagram showing the arrangement of another activity state analysis device according to the third embodiment of the present invention;

Fig. 12 is a block diagram showing the arrangement of an activity state analysis device according to the fourth embodiment of the present invention;

Fig. 13A is an explanatory view for explaining an effect obtained by the activity state analysis device according to the fourth embodiment of the present invention;

Fig. 13B is an explanatory view for explaining an effect obtained by the activity state analysis device according to the fourth embodiment of the present invention;

Fig. 13C is an explanatory view for explaining an effect obtained by the activity state analysis device according to the fourth embodiment of the present invention;

Fig. 14 is a block diagram showing the arrangement of an activity state analysis device according to the fifth embodiment of the present invention;

Fig. 15A is an explanatory view for explaining an effect obtained by the activity state analysis device according to the fifth embodiment of the present invention;

Fig. 15B is an explanatory view for explaining an effect obtained by the activity state analysis device according to the fifth embodiment of the present invention;

Fig. 15C is an explanatory view for explaining an effect obtained by the activity state analysis device according to the fifth embodiment of the present invention; and

Fig. 16 is a block diagram showing the arrangement of an activity state analysis device according to the sixth embodiment of the present invention.

Best Mode for Carrying Out the Invention

The embodiments of the present invention will now be described with reference to the accompanying drawings.

[First Embodiment]

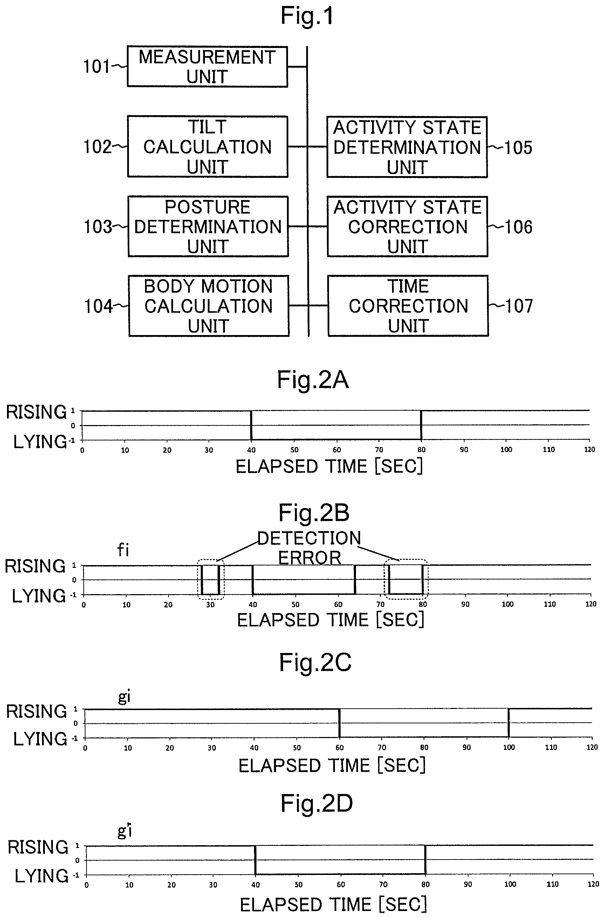

The arrangement of an activity state analysis device according to the first embodiment of the present invention will be described first with reference to Fig. 1. The activity state analysis device includes a measurement unit 101, a tilt calculation unit 102, a posture determination unit 103, a body motion calculation unit 104, an activity state determination unit 105, an activity state correction unit 106, and a time correction unit 107.

The measurement unit 101 is formed by a well-known acceleration sensor and attached to a measurement target person to measure acceleration. The measurement unit 101 periodically measures accelerations in the three axis directions along X-, Y-, and Z-axes that are orthogonal to each other at a sampling rate of, for example, 25 Hz, thereby obtaining the time series of the accelerations.

The tilt calculation unit 102 obtains the angle of the tilt of the upper part of the body of the measurement target person based on the accelerations measured by the measurement unit 101. For example, the tilt calculation unit 102 calculates $\theta$ and $\phi$ by equations below as the tilts of the measurement unit 101 with respect to the gravitational accelerations of the accelerations measured by the measurement unit 101.

Here, $\theta$ ($-90 \le \theta < 270$) is the tilt of the Z-axis of the acceleration sensor with respect to the vertical direction, and $\phi$ ($-90 \le \phi < 270$) is the tilt of the X-axis of the acceleration sensor with respect to the vertical direction. The unit is the degree [degrees].

$$\theta = \frac{180}{\pi} \cos^{-1}\left(\frac{A_z}{\sqrt{A_x^2 + A_y^2 + A_z^2}}\right) + 90 \cdots \left(for\ A_y \geq 0\right)$$

$$\theta = -\frac{180}{\pi} \cos^{-1}\left(\frac{A_z}{\sqrt{A_x^2 + A_y^2 + A_z^2}}\right) + 90 \cdots \left(for\ A_y < 0\right)$$

$$\cdots (1)$$

$$\emptyset = \frac{180}{\pi} \cos^{-1}\left(\frac{A_x}{\sqrt{A_x^2 + A_y^2 + A_z^2}}\right) + 90 \cdots \left(for\ A_y \geq 0\right)$$

$$\emptyset = -\frac{180}{\pi} \cos^{-1}\left(\frac{A_x}{\sqrt{A_x^2 + A_y^2 + A_z^2}}\right) + 90 \cdots \left(for\ A_y < 0\right)$$

$$\cdots (2)$$

where $A_x$, $A_y$, and $A_z$ are accelerations in the X-, Y-, and Z-axis directions measured by the measurement unit 101, and the unit is the gravitational acceleration G (1.0 G $\approx$ 9.8 m/s$^2$). In each of equations (1) and (2), the ratio of the measurement value of a single axis with respect to the magnitude (norm) of the composite vector of the accelerations in the X-, Y-, and Z-axis directions measured by the measurement unit 101 is obtained, and the inverse function of the cosine is further obtained, thereby calculating the tilt of the measurement unit 101 as a value having the dimension of the angle.

As $A_x$, $A_y$, and $A_z$ in equations (1) and (2), the output values of the measurement unit 101 may directly be substituted. Alternatively, values obtained by applying a low-pass filter (for example, an FIR filter or a moving average filter) for smoothing to the output values may be used.

The posture determination unit 103 determines the posture of the measurement target person based on the tilts of the measurement unit 101 obtained by the tilt calculation unit 102. For example, the posture determination unit 103 compares the values $\theta$ and $\phi$ calculated by equations (1) and (2) with thresholds, thereby determining the posture. Since the tilts of the measurement unit 101 reflect the tilts of the upper part of the body of the measurement target person who wears the measurement unit 101, the posture of the measurement target person can be calculated from the tilts of the measurement unit 101.

The posture determination unit 103 determines the posture of the measurement target person based on, for example, the following classification.

(i) Standing position (upright): when $30 \leq \theta < 140$.

(ii) Standing position (inverted): when $\theta < -40$ or $220 < \theta$.

(iii) Lying position (the left side of the body is located on the upper side): when ($\phi \leq -50$, or $230 < \phi$) and ($-40 \leq \theta < 30$), or when ($\phi \leq -50$, or $230 < \phi$) and ($140 < \theta < 220$).

(iv) Lying position (the right side of the body is located on the upper side): when ($50 < \phi < 130$) and ($-40 \leq \theta < 30$), or when ($50 < \phi < 130$) and ($140 < \theta < 220$) .

(v) Lying position (lying with the face up): when ($130 \leq \phi \leq 230$) and ($-40 \leq \theta < 30$), or when ($130 \leq \phi \leq 230$) and ($140 < \theta < 220$).

(vi) Lying position (lying with the face down): when ($-50 \leq \phi \leq 50$) and ($-40 \leq \theta < 30$), or when ($-50 \leq \phi \leq 50$) and ($140 < \theta < 220$).

In the above-described classification conditions, the thresholds used to determine the posture do not match the angles (-45, 45, 135, 225) that divide the quadrants of a unit circle on two-dimensional coordinates. This is because when a human body stands upright, the range of motion of the back is assumed to be large in a case of, for example, bending forward to look into something or bending backward to look up. In the above-described example, according to the reality of measurement values obtained by the measurement unit 101 set on the trunk, a wide calculation region is ensured for the upright position as compared to the measurement values for the lying positions. This method is the same as the method of non-patent literature 1.

The above-described definitions (i) to (vi) of calculation are set (stored) in the posture determination unit 103 as a table of θ and φ as shown in Table 1 below.

[Table 1]

| | φ(−90 ≤ φ < 270) | | | | |
| --- | --- | --- | --- | --- | --- |
| | −50 | 50 | 130 | 230 | |
| −40 | inverted | inverted | inverted | inverted | inverted |
| 30 | left side of body up | lying with face down | right side of body up | lying with face up | left side of body up |
| 140 | upright | upright | upright | upright | upright |
| 220 | left side of body up | lying with face down | right side of body up | lying with face up | left side of body up |
| | inverted | inverted | inverted | inverted | inverted |

θ(−90 ≤ θ < 270)

The body motion calculation unit 104 obtains the magnitude of a body motion representing the magnitude (intensity) of an action of the measurement target person based on the accelerations measured by the measurement unit 101.

The activity state determination unit 105 determines, based on the posture determined by the posture determination unit 103 and the magnitude of the body motion calculated by the body motion calculation unit 104, whether the activity state of the measurement target person is a first state or a second state different from the first state. The first state is, for example, a state in which the measurement target person gets up (rising state). In addition, the second state is, for example, a state in which the measurement target person is lying in the bed (lying state). A case in which the activity state determination unit 105 time-serially obtains the activity state representing the rising state as the first state or the lying state as the second state will be described below as an example.

For example, the activity state determination unit 105 determines the activity state based on the conditions to be described below. A case in which as the grasping of the mid and long-term activity tendency of the measurement target person, the total time (24 hrs) of one day is identified into a rising period that is a period during which the measurement target person is up and a lying period that is a period during which the measurement target person is lying in the bed will be described below.

First, if the posture is determined as a standing position by the classifications (i) and (ii), the activity state is classified as rising. On the other hand, if the posture is calculated as a lying position by the classifications (iii) to (vi), the accuracy of determination needs to be raised because, for example, the posture may erroneously be determined as lying face down even in a case of bending forward in the standing position. To improve the accuracy, the lying position and the rising are classified in consideration of the magnitude (intensity) of the body motion obtained by the body motion calculation unit 104.

In the body motion calculation unit 104, the variance value of time-series data of an acceleration measured by the measurement unit 101 is used as the index of the magnitude of the body motion by referring to the method described in patent literature 1. Let i be a positive integer to be incremented by one by each sampling of acceleration data from the measurement start time (i = 1, 2,....) For example, let $a_i$ be the value of an acceleration norm obtained by the measurement unit 101 at an ith sampling time $t_i$, time-series acceleration data of 50 points be the population, $A_i$ be the average, and $S_i^2$ be the variance value. In this case, $A_i$ and $S_i^2$ are represented as follows

$$A_i = \frac{(a_{i-24} + \cdots + a_{i-1} + a_i + a_{i+1} + \cdots + a_{i+25})}{50}$$

$$S_i^2 = \frac{1}{50} \sum_{k=-24}^{25} (a_{i+k} - A_i)^2$$

If the variance value $S_i^2$ exceeds a predetermined magnitude, it can be determined that a conscious body motion has occurred, and the measurement target person has temporarily taken the posture of bending forward in accordance with the purpose at a high possibility. For this reason, in this case, even in the classifications (iii) to (vi), it is determined that the measurement target person is in the rising state, and the state is classified as rising. For example, if (iii) to (vi) are satisfied, and $S_i^2 \geq 0.01$ is satisfied, the state is determined as the rising state. On the other hand, if $S_i^2 \geq 0.01$ is not satisfied, and (iii) to (vi) are satisfied, the state is determined as the lying state.

Based on the rising state and the lying state determined in the above-described way, the activity state determination unit 105 performs an operation by a state function $f_i$ to be described next, and outputs the state at the ith sampling time ti as 1 that represents the rising state or -1 that represents the lying state.

$f_i$ = 1 (when the state is determined as rising)
$f_i$ = -1 (when the state is determined as lying)

If, in the time series of the activity state obtained by the activity state determination unit 105, the activity state transitions from one state of the rising state and the lying state to the other state, and the other state continues for a predetermined time defined in advance, the activity state correction unit 106 determines that the activity state has transitioned from the one state to the other state. If the other state does not continue for the predetermined time, the activity state correction unit 106 determines that the activity state has not transitioned from the one state to the other state.

A determination error may remain in the lying state determined by the above-described activity state determination unit 105. For example, in a case in which the body motion unconsciously exceeds a predetermined value by rollover or the like, a determination error may occur in the determination of the activity state determination unit 105. Suppressing of the determination error is performed by the activity state correction unit 106. As the method, the determination error is suppressed by providing a predetermined time defined in advance as a dead band at the time of switching of posture determination (state transition) by referring to the past history. This can further improve the accuracy of activity state determination.

By the following method, for example, the activity state correction unit 106 calculates a function gi from the value of the state function $f_i$ output from the activity state determination unit 105 using the state functions from the state function at the ith sampling time to the sampling time i-$\alpha$ back by $\alpha$ from the sampling time. Here, $\alpha$ is 0 or a positive integer.

$g_i$ = $f_i$ (when $f_i$ = $f_{i-1}$ = ... = $f_{i-\alpha}$ holds)
$g_i$ = $g_{i-1}$ (when $f_i$ = $f_{i-1}$ = ... = $f_{i-\alpha}$ does not hold, that is, when at least one of the equal signs does not hold from $f_i$ to $f_{i-\alpha}$)

For example, assume that $g_1$ = $f_1$, and $\alpha$ = 500. Note that if the number of data is less than 500, $\alpha$ is set to the same value as the number of data. When the sampling rate in the measurement unit 101 is 25 Hz, gi = $f_i$ holds after the elapse of 20 [sec] (= 500/25) at the shortest from the start of the measurement. When $\alpha$ = 500, switching of $f_i$ is not reflected on $g_i$ unless 20 sec do not elapse in the state after the switching. As described above, the activity state correction unit 106 sets the above-described time (20 sec) to the predetermined time. If the activity state after the transition continues for the predetermined time, the activity state correction unit 106 determines that the transition of the activity state has been done.

When the activity state correction unit 106 determines that the activity state has transitioned from one state to the other state, the time correction unit 107 sets the time set back by the predetermined time from the time at which the activity state correction unit 106 has determined that the activity state has transitioned from the one state to the other state, as the transition time at which the activity state has transitioned from the one state to the other state.

This is because in the activity state corrected by the activity state correction unit 106, the time of transition between the rising state and the lying state shifts (delays) by the predetermined time. In the above-described example, the time of transition between the rising state and the lying state delays by 20 sec. Hence, the time correction unit 107 sets the predetermined time to the delay time, and corrects the output of the activity state correction unit 106. The time correction unit 107 outputs, for example, $g'_i$ = $g_{i+\alpha}$ obtained by advancing the time stamp of the time-serially obtained output $g_i$ of the activity state correction unit 106 by 20 sec.

Effects according to the first embodiment will be described next with reference to Figs. 2A, 2B, 2C, and 2D. Figs. 2A and 2B show the time-series change of $f_i$ representing the activity state. Figs. 2C and 2D show the time-series change of $g_i$ representing the corrected activity state.

Fig. 2A shows correct $f_i$ that should be the correct answer. 1 represents the rising state, and -1 represents the lying state. In actual measurement, however, a determination error occurs, as shown in Fig. 2B. When the activity state correction unit 106 corrects the time-series change of $f_i$ shown in Fig. 2B, in which the determination error occurs, $g_i$ in which the determination error is corrected is obtained, as shown in Fig. 2C.

However, in the time-series change of $g_i$ shown in Fig. 2C, the time of switching between the rising state and the lying state delays by the predetermined time. When this delay is corrected by the time correction unit 107, the time-series change of $g'_i$, which is the same as the time-series change of $f_i$ shown in Fig. 2A, is obtained as shown in Fig. 2D, and an activity state according to the reality can be obtained, as is apparent. Note that if gi is not updated due to the end of measurement or the like, a period during which g'i is not obtained occurs. In this case, as a substitute value, g'i = gi is set to prevent a data loss.

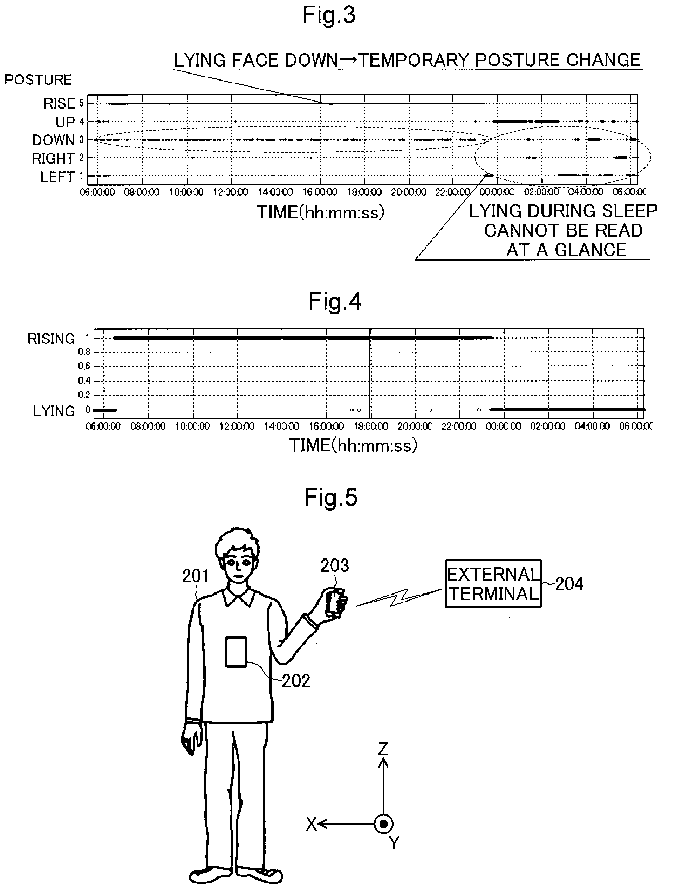

An effect of the first embodiment obtained by executing measurement for 24 hrs will be described next with reference to Figs. 3 and 4. In g'i, -1 is converted into 0 to save bits used for calculation. In the output of the activity state determination unit 105 corresponding to posture calculation performed in the conventional technique, determination values are dissipated due to instantaneous posture variations, as shown in Fig. 3, and it is difficult for a general user unfamiliar to handling of an electronic device to understand the result. Note that referring to Fig. 3, 1 on the ordinate represents a state in which the left side of the body faces up. 2 on the ordinate of Fig. 3 represents a state in which the right side of the body faces up. 3 on the ordinate of Fig. 3 represents a lying face down state. 4 on the ordinate of Fig. 3 represents a lying face up state.

On the other hand, according to the first embodiment, since correction is performed by the activity state correction unit 106 and the time correction unit 107, an output in which the rising state and the lying state can be identified at a glance can be obtained, as shown in Fig. 4, and the state can easily be grasped.

According to the first embodiment, correction using a dead band in addition to the magnitude of the body motion and correction of a delay caused by the correction are performed, thereby removing a disturbance caused by an instantaneous change of the position. It is therefore possible to identify 24 hrs the measurement target person spends into the rising period and the lying period, and appropriately grasp the activity state of the measurement target person.

An activity state analysis method using the activity state analysis device according to the above-described first embodiment includes the following steps. First, the measurement unit 101 measures the acceleration in an action of the measurement target person (first step). For example, the measurement unit 101 periodically measures accelerations in the three directions along the X-, Y-, and Z-axes that are orthogonal to each other at a sampling rate of, for example, 25 Hz, thereby obtaining the time series of the accelerations. Next, the tilt calculation unit 102 obtains the angle of the tilt of the upper part of the body of the measurement target person based on the accelerations measured by the measurement unit 101 (first step) (second step). Next, the posture determination unit 103 determines the posture of the measurement target person based on the angle of tilt obtained by the tilt calculation unit 102 (second step) (third step).

Then, the body motion calculation unit 104 obtains the magnitude of the body motion of the measurement target person based on the accelerations measured by the measurement unit 101 (first step) (fourth step). Next, the activity state determination unit 105 determines, based on the posture determined by the posture determination unit 103 (third step) and the magnitude of the body motion calculated by the body motion calculation unit 104 (fourth step), whether the activity state of the measurement target person is the rising state (first state) or the lying state (second state) (fifth step).

If, in the time series of the activity state obtained by the activity state determination unit 105 (fifth step), the activity state transitions from one state of the rising state and the lying state to the other state, and the other state continues for a predetermined time defined in advance, the activity state correction unit 106 determines that the activity state has transitioned from the one state to the other state. If the other state does not continue for the predetermined time, the activity state correction unit 106 determines that the activity state has not transitioned from the one state to the other state (sixth step).

Next, when the activity state correction unit 106 (sixth step) determines that the activity state has transitioned from one state to the other state, the time set back by the predetermined time from the time at which it is determined that the activity state has transitioned from the one state to the other state in the sixth step is set as the transition time at which the activity state has transitioned from the one state to the other state (seventh step).

Note that the activity state analysis device according to the above-described first embodiment is a computer device including a CPU (Central Processing Unit), a main storage device, an external storage device, a network connection device, and the like. The above-described functions are implemented when the CPU operates by a program loaded into the main storage device. Additionally, the functions may be distributed to a plurality of computer devices.

A system using the activity state analysis device according to the first embodiment will be described next. For example, as shown in Fig. 5, a sensor terminal 202 is attached to the trunk of a measurement target person 201, and a result measured by the sensor terminal 202 is transmitted to an external terminal 204 via a relay terminal 203. A detection direction X of an acceleration in the sensor terminal 202 is arranged in parallel to the left-to-right direction of the body of the measurement target person 201. A detection direction Y of an acceleration in the sensor terminal 202 is arranged in parallel to the front-and-rear direction of the body of the measurement target person 201. A detection direction Z of an acceleration in the sensor terminal 202 is arranged in parallel to the up-and-down direction of the body of the measurement target person 201.

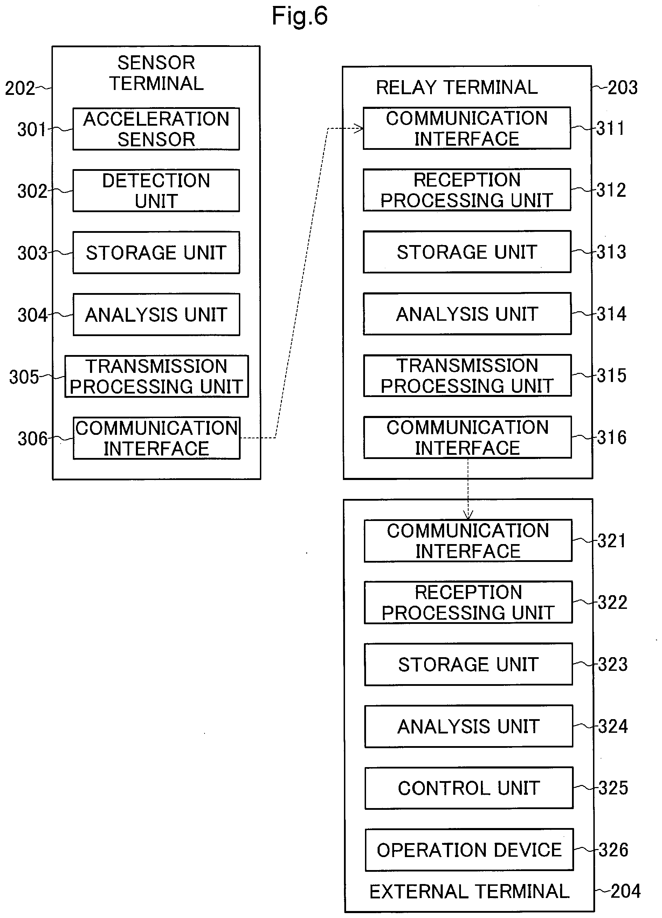

As shown in Fig. 6, the sensor terminal 202 includes an acceleration sensor 301, a detection unit 302, a storage unit 303, an analysis unit 304, a transmission processing unit 305, and a communication interface 306. The relay terminal 203 includes a communication interface 311, a reception processing unit 312, a storage unit 313, an analysis unit 314, a transmission processing unit 315, and a communication interface 316. The external terminal 204 includes a communication interface 321, a reception processing unit 322, a storage unit 323, an analysis unit 324, a control unit 325, and an operation device 326.

The acceleration sensor 301 measures accelerations in the three directions along the X-, Y-, and Z-axes that are orthogonal to each other. The detection unit 302 converts the analog acceleration signal measured by the acceleration sensor 301 into digital acceleration data at a predetermined sampling rate and outputs it. The measurement unit 101 according to the above-described embodiment corresponds to the acceleration sensor 301. The storage unit 303 stores the acceleration data digitized by the detection unit 302. The analysis unit 304 obtains an activity state based on the acceleration data stored in the storage unit 303, and the like. The tilt calculation unit 102, the body motion calculation unit 104, the posture determination unit 103, the activity state determination unit 105, the activity state correction unit 106, and the time correction unit 107 according to the above-described embodiment are included in the analysis unit 304.

The transmission processing unit 305 transmits the acceleration data stored in the storage unit 303, and the like to the relay terminal 203 via the communication interface 306. The communication interface 306 is formed by an operation interface and an antenna corresponding to a wireless data communication standard such as LTE (Long Term Evolution), third generation mobile communication system, wireless LAN (Local Area Network), or Bluetooth®.

The relay terminal 203 is formed from the communication interface 311 that receives data transmitted from the sensor terminal 202, the reception processing unit 312, the storage unit 313, the analysis unit 314, the transmission processing unit 315, and the communication interface 316 that transmits data to the external terminal 204.

The external terminal 204 includes the communication interface 321 that receives data transmitted from the relay terminal 203, the reception processing unit 322, the storage unit 323, the analysis unit 324, and the control unit 325 that instructs an operation instruction to the operation device 326 that operates based on analyzed data.

Based on information stored in the storage unit 323, the control unit 325 causes the operation device 326 to execute an operation to assist the measurement target person.

The operation device 326 is a video output device (a monitor or the like), a voice output device (a speaker, a musical instrument, or the like), a light source (an LED (Light Emitting Diode) or an electric bulb), an actuator (a vibrator, a robot arm, or an electrotherapeutic instrument), a cooling/heating device (a heater or a Peltier element), or the like.

Not all of the analysis unit 304 of the sensor terminal 202, the analysis unit 314 of the relay terminal 203, and the analysis unit 324 of the external terminal 204 need be arranged, and only one or two of them may be provided. In addition, analysis processing may be distributed to the analysis unit 304, the analysis unit 314, and the analysis unit 324 in accordance with the steps of the activity state analysis method according to the first embodiment.

The result by g'i shown in Fig. 2D is presented by the operation device 326 of the external terminal 204. In addition, the operation device 326 not only presents the above-described activity state analysis result but also makes a signal by a sound, vibration, contact, heat, coldness, or the like to the measurement target person or the user of the external terminal 204 when switching of the activity state has occurred, thereby notifying the change of the activity state.

[Second Embodiment]

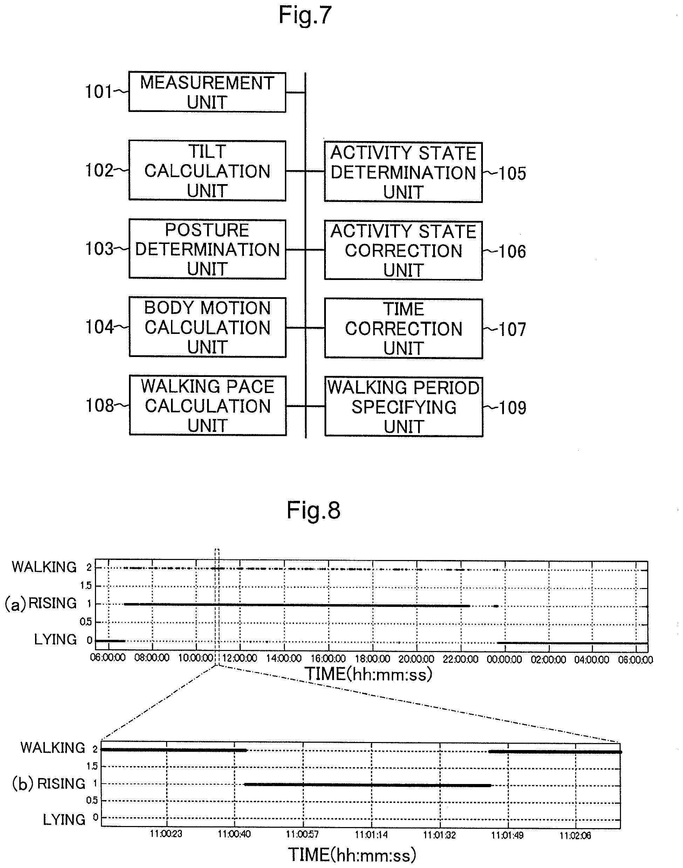

The second embodiment of the present invention will be described next with reference to Fig. 7. Fig. 7 is a block diagram showing the arrangement of an activity state analysis device according to the second embodiment of the present invention. The activity state analysis device includes a measurement unit 101, a tilt calculation unit 102, a posture determination unit 103, a body motion calculation unit 104, an activity state determination unit 105, an activity state correction unit 106, and a time correction unit 107. These components are the same as in the above-described first embodiment.

The activity state analysis device according to the second embodiment includes a walking pace calculation unit 108 and a walking period specifying unit 109 in addition to the above-described components.

The walking pace calculation unit 108 obtains the walking pace of a measurement target person based on the acceleration measured by the measurement unit 101. The walking pace calculation unit 108 obtains the walking pace of the measurement target person based on the time-series data of the acceleration measured by the measurement unit 101. For example, the acceleration at a standstill is about 1 G. As a feature, the time-rate change of the acceleration in a walking state or a running state exhibits a vibration waveform with respect to 1 G as the center. Using this feature, a lower threshold and an upper threshold are set to 0.9 G and 1.1 G, respectively. A timing at which the acceleration is less than the lower threshold or a timing at which the acceleration is more than the upper threshold is detected from the obtained time-series data of the acceleration. If two timing detections occur within, for example, 1 sec, it is determined that a walking action has occurred, and one step is counted. In addition, when conversion is performed to obtain the number of times of counting that has occurred in a unit time, the walking pace (spm) can be obtained (see patent literature 1).

Based on the walking pace of the measurement target person obtained by the walking pace calculation unit 108, the walking period specifying unit 109 specifies the period of the walking state in which the measurement target person was walking in the period of the rising state (first state) defined by the transition time corrected by the time correction unit 107. For example, the walking period specifying unit 109 specifies, as the period of the walking state, a period during which the walking pace measured by the walking pace calculation unit 108 exceeds 15 spm in the period of rising. Note that in the second embodiment, running is included in walking.

In an activity state analysis method according to the second embodiment, the following steps are added to the steps of the activity state analysis method using the activity state analysis device according to the above-described first embodiment.

First, the walking pace calculation unit 108 obtains the walking pace of the measurement target person based on the acceleration measured by the measurement unit 101 (first step) (eighth step). Next, the walking period specifying unit 109 specifies, based on the walking pace of the measurement target person obtained by the walking pace calculation unit 108 (eighth step), the period of the walking state in which the measurement target person was walking in the period of the rising state defined by the transition time corrected by the time correction unit 107 (seventh step) (ninth step).

According to the above-described second embodiment, the activity state is time-serially obtained, as shown in Fig. 8. As shown in Fig. 8, the walking period specifying unit 109 outputs 2 as the walking state. Note that (b) of Fig. 8 shows an enlarged view of a part in (a) of Fig. 8. According to the second embodiment, the walking state is independently shown, as shown in Fig. 8. As described above, according to the second embodiment, it is possible to grasp, at a glance, for example, a period in which the measurement target person is not only rising but also the walking frequency is high.

[Third Embodiment]

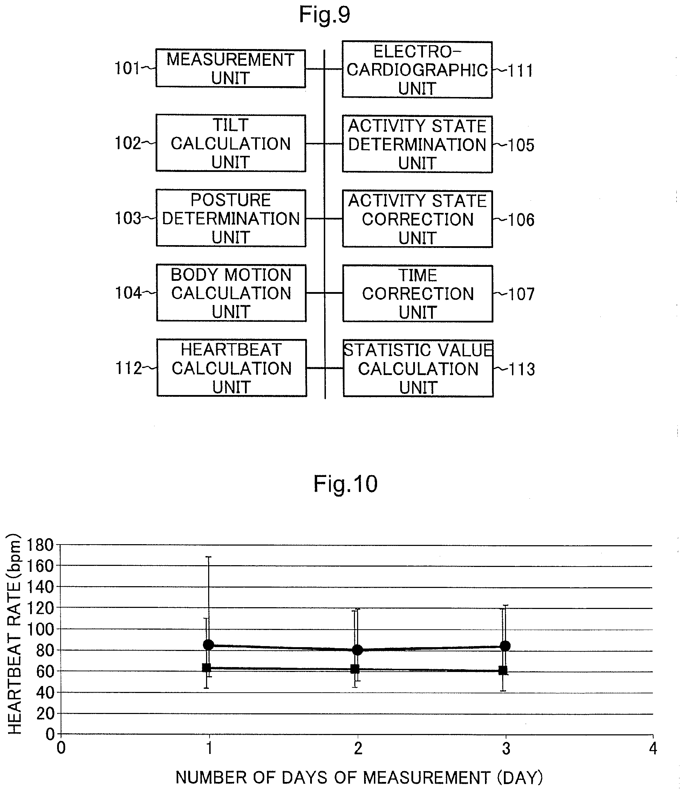

The third embodiment of the present invention will be described next with reference to Fig. 9. Fig. 9 is a block diagram showing the arrangement of an activity state analysis device according to the third embodiment of the present invention. The activity state analysis device includes a measurement unit 101, a tilt calculation unit 102, a posture determination unit 103, a body motion calculation unit 104, an activity state determination unit 105, an activity state correction unit 106, and a time correction unit 107. These components are the same as in the above-described first embodiment.

The activity state analysis device according to the third embodiment includes an electrocardiographic unit 111, a heartbeat calculation unit 112, and a statistic value calculation unit 113 in addition to the above-described components. The electrocardiographic unit 111 and the heartbeat calculation unit 112 form a physical information measurement unit.

The electrocardiographic unit 111 measures the electrical information (cardiac potential) of the heart of a measurement target person. The heartbeat calculation unit 112 calculates at least one of a heartbeat interval (RRI) and a heart rate from the measurement value measured by the electrocardiographic unit 111. In the third embodiment, the heartbeat interval and the heart rate are obtained as physical information.

The statistic value calculation unit 113 obtains a statistic value including at least one of the average value, the median, the maximum value, the minimum value, the standard deviation, the 75% level value, and the 250 level value of at least one of the heartbeat interval and the heart rate obtained by the heartbeat calculation unit 112. The statistic value calculation unit 113 obtains the statistic value based on an activity state defined by the transition time corrected by the time correction unit 107. The statistic value calculation unit 113 obtains the statistic value for each of states such as lying and rising in the activity state corrected by the time correction unit 107.

In an activity state analysis method according to the third embodiment, the following steps are added to the steps of the activity state analysis method using the activity state analysis device according to the above-described first embodiment.

First, the physical information of the measurement target person is measured (10th step). More specifically, the electrocardiographic unit 111 measures the electrical activity of the heart of the measurement target person, and the heartbeat calculation unit 112 calculates, as physical information, at least one of the heartbeat interval and the heart rate from the measurement value measured by the electrocardiographic unit 111. Next, based on the activity state defined by the transition time corrected in the seventh step, the statistic value calculation unit 113 obtains a statistic value including at least one of the average value, the median, the maximum value, the minimum value, the standard deviation, the 75% level value, and the 25% level value of at least one of the heartbeat interval and the heart rate obtained by the heartbeat calculation unit 112 (11th step).

According to the above-described third embodiment, the statistic value can be grasped time-serially, as shown in Fig. 10. Fig. 10 shows a rising state (full circles) and a lying state (full squares) as activity states. Fig. 10 also shows the statistic values of the heart rate in these states. As the statistic value, the average vale is indicated by a point of a full circle or a full square. The error bar on the upper side represents the maximum value, and the error bar on the lower side represents the minimum value.

In this way, analysis based on the activity state can be performed each day. When the measurement is performed for a long term, it is possible to easily grasp, based on the activity state, the sign of recovery of a disease, a load given to the body by a seasonal change, a change in conditions caused by an exercise habit, or the like. In addition, when the above-described result is fed back, it can contribute to lifestyle investigations or improvement of lifestyle.

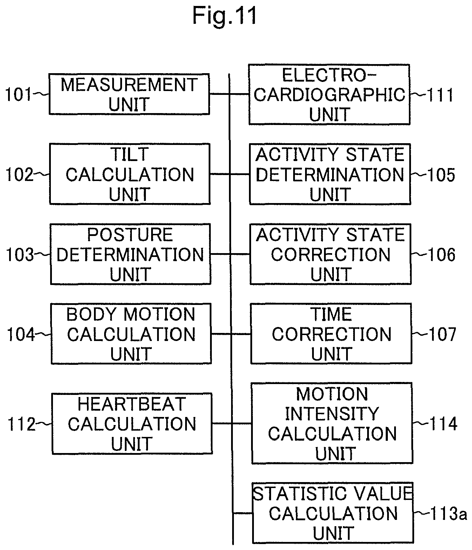

A motion intensity may be used in place of the heart rate. For example, as shown in Fig. 11, a motion intensity calculation unit 114 is provided to calculate a motion intensity from the heart rate calculated by the heartbeat calculation unit 112. The motion intensity is obtained by (measured heart rate - resting heart rate of measurement target person) ÷ (maximum heart rate of measurement target person - resting heart rate) (see non-patent literature 3). Note that the maximum heart rate of the measurement target person is set to 220 - (age of measurement target person). In addition, the resting heart rate of the measurement target person is set to 60. The maximum heart rate of the measurement target person and the resting heart rate of the measurement target person may be actually measured in advance. For example, as the resting heart rate, the heart rate during a period in which the measurement target person is sitting quietly is used. Alternatively, the average value, the median, or the minimum value may be obtained from the heart rate during the lying period of the previous day or during the lying period of the previous night (0:00 to 5:00) and used as the resting heart rate.

A statistic value calculation unit 113a obtains a statistic value including at least one of the average value, the median, the maximum value, the minimum value, the standard deviation, the 75% level value, and the 25% level value of the motion intensity obtained by the motion intensity calculation unit 114. The statistic value calculation unit 113a obtains the above-described statistic value based on the activity state defined by the transition time corrected by the time correction unit 107. The statistic value calculation unit 113a obtains the statistic value for each of states such as lying and rising in the activity state corrected by the time correction unit 107.

In the activity state analysis method in this case, the following steps are added to the steps of the activity state analysis method using the activity state analysis device according to the above-described first embodiment.

First, the electrocardiographic unit 111 measures the electrical activity of the heart of the measurement target person (10th step). Next, the heartbeat calculation unit 112 calculates the heart rate from the measurement value measured by the electrocardiographic unit 111 (10th step) (11th step). Next, the motion intensity calculation unit 114 calculates the motion intensity based in the heart rate obtained by the heartbeat calculation unit 112 (11th step) (12th step). Next, the statistic value calculation unit 113a obtains a statistic value including at least one of the average value, the median, the maximum value, the minimum value, the standard deviation, the 75% level value, and the 250 level value of the motion intensity obtained by the motion intensity calculation unit 114 (12th step) based on the activity state defined by the transition time corrected in the seventh step (13th step).

Even when the statistic value is time-serially grasped for the motion intensity in this way, analysis based on the activity state can be performed each day, as described above. When the measurement is performed for a long term, it is possible to easily grasp, based on the activity state, the sign of recovery of a disease, a load given to the body by a seasonal change, a change in conditions caused by an exercise habit, or the like. In addition, when the above-described result is fed back, it can contribute to lifestyle investigations or improvement of lifestyle.

In addition, the physical information may be a heart rate measured by a pulse measurement unit that measures the physical contraction of the pulse of the measurement target person. The physical information may be a respiratory state measured by an impedance measurement unit that measures the impedance of the body of the measurement target person, thereby measuring the respiratory state. The physical information may be a blood pressure measured by a blood pressure measurement unit that measures the blood pressure of the measurement target person. The physical information may be a body temperature measured by a body temperature measurement unit that measures the body temperature of the measurement target person. The physical information may be a muscle potential measured by an electromyographic unit that measures the muscle potential of the measurement target person. The physical information may be a body weight measured by a body weight measurement unit that measures the body weight of the measurement target person.

In addition, the physical information may be a calorie consumption measured by a calorie measurement unit that measures the calorie consumption of the measurement target person. The physical information may be an activity or sleeping action measured by an activity measurement unit that measures the activity or sleeping action of the measurement target person by an electroencephalograph or an acceleration sensor. The physical information may be sweating measured by a sweating measurement unit that measures the sweating of the measurement target person.

[Fourth Embodiment]

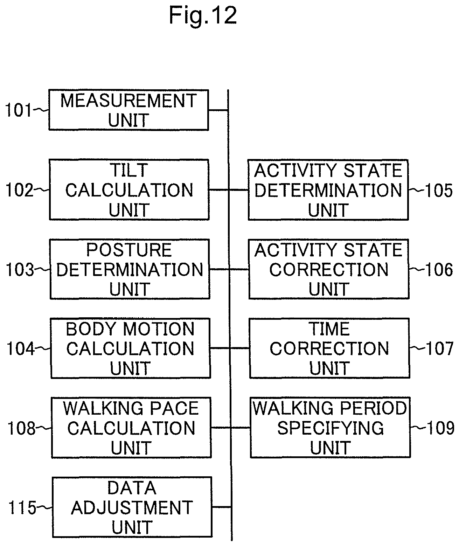

The fourth embodiment of the present invention will be described next with reference to Fig. 12. An activity state analysis device includes a measurement unit 101, a tilt calculation unit 102, a posture determination unit 103, a body motion calculation unit 104, an activity state determination unit 105, an activity state correction unit 106, a time correction unit 107, a walking pace calculation unit 108, and a walking period specifying unit 109. These components are the same as in the above-described second embodiment.

The activity state analysis device according to the fourth embodiment includes a data adjustment unit 115 in addition to the above-described components. The data adjustment unit 115 downsamples the time-serially obtained data of the activity state including a rising state (first state) and a lying state (second state), which are time-serially obtained, while assigning a priority to each state of the first state and the second state. In the fourth embodiment, a walking state is also included in the activity state. The data of the activity state is a time series whose time is corrected by the time correction unit 107, and is formed by the data of the rising state and the lying state determined by the activity state determination unit 105 and corrected by the activity state correction unit 106 and the walking state specified by the walking period specifying unit 109. When downsampling is performed by the data adjustment unit 115, the number of data of the activity state can be reduced by thinning.

The data adjustment unit 115 downsamples, for example, data per sec at an interval of 1 min. In this downsampling processing, the data adjustment unit 115 assigns a priority to each of walking, rising, and lying, and gives a high priority of walking.

The data adjustment unit 115 holds the data of the activity state obtained at an interval of, for example, 1 sec for 1 min (60 points). Next, if walking is determined continuously for 6 sec (6 points) in the period of 1 min of holding, the data adjustment unit 115 determines the data of one point after the downsampling as walking. Note that the continuation time for the determination is not limited to 6 sec. However, 6 sec is a time necessary for an able-bodied person to walk about 10 steps, or for an elderly patient to walk about six steps. This is appropriate because walking shifted to a side a little can be excluded. If the walking state is not obtained continuously for 6 sec, the data adjustment unit 115 employs a longer one of the rising and lying included in 1 min as the data of 1 point after downsampling. If the rising and lying have the same point (time), the data adjustment unit 115 gives a priority to rising.

In an activity state analysis method according to the fourth embodiment, the following step is added to the steps of the activity state analysis method using the activity state analysis device according to the above-described second embodiment. The data adjustment unit 115 downsamples the time-serially obtained data of the activity state.

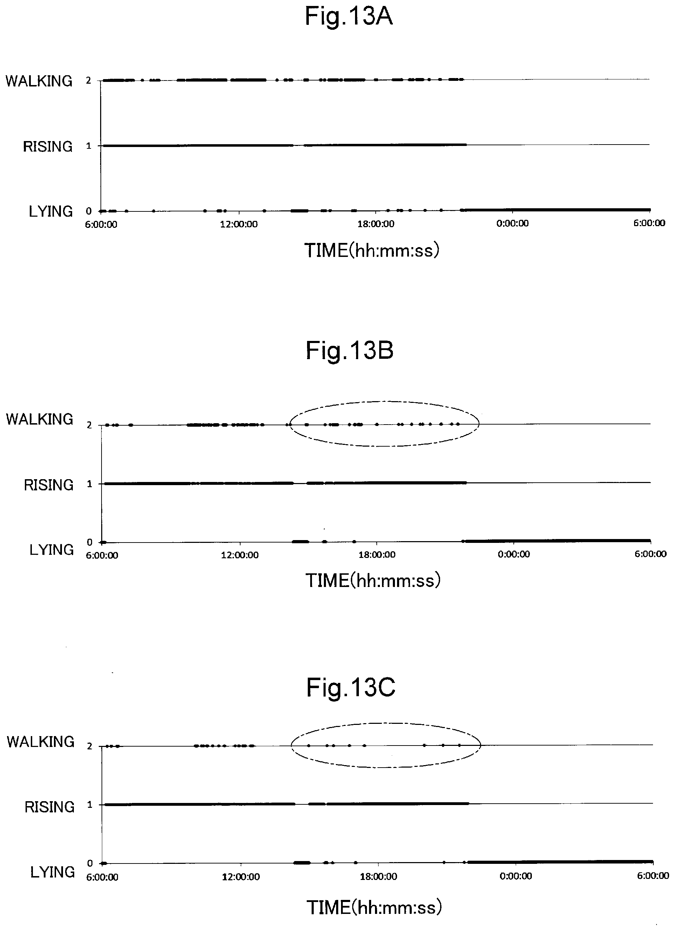

Effects according to the fourth embodiment will be described with reference to Figs. 13A, 13B, and 13C. Fig. 13A shows the time-series data of an activity state before downsampling. Fig. 13B shows the result of downsampling at an interval of 1 min by the data adjustment unit 115. Fig. 13C shows a downsampling result obtained when data is extracted mechanically once in 60 times from the data per sec.

Walking requires the physical strength of the measurement target person, unlike rising and lying. Hence, the walking state is poor in continuity. For this reason, if downsampling is mechanically performed, the information of walking may be lost, as shown in Fig. 13C. In the result shown in Fig. 13C, the walking state is lost, as is apparent from comparison with Fig. 13A.

On the other hand, according to the fourth embodiment, downsampling is performed while assigning a priority to each state. As shown in Fig. 13B, downsampling can be performed in a state in which the loss of the walking state is reduced. As a result, according to the fourth embodiment, it is possible to efficiently use the time-serially obtained data of the activity state and avoid the resource of a CPU or a memory from being oppressed at the time of data presentation.

[Fifth Embodiment]

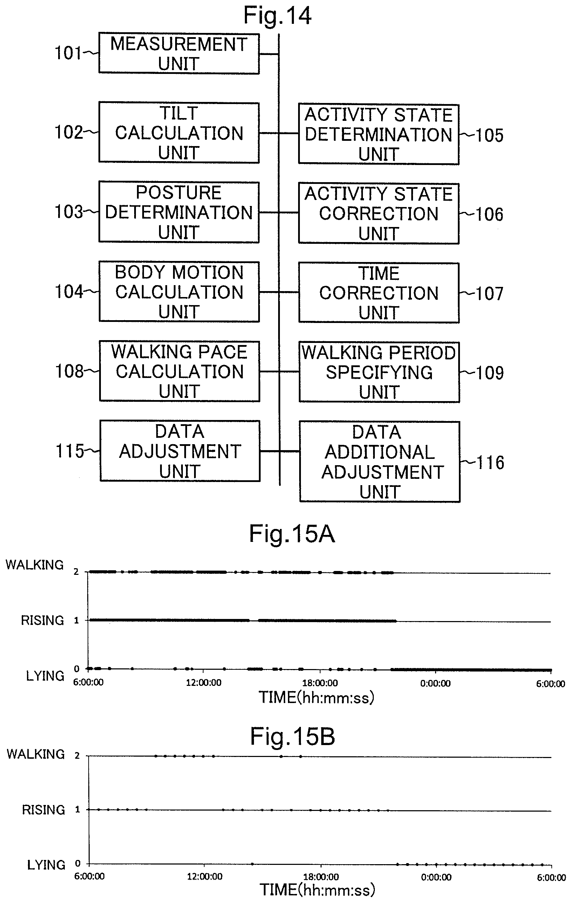

The fifth embodiment of the present invention will be described next with reference to Fig. 14. An activity state analysis device includes a measurement unit 101, a tilt calculation unit 102, a posture determination unit 103, a body motion calculation unit 104, an activity state determination unit 105, an activity state correction unit 106, a time correction unit 107, a walking pace calculation unit 108, a walking period specifying unit 109, and a data adjustment unit 115. These components are the same as in the above-described fourth embodiment.

The activity state analysis device according to the fifth embodiment includes a data additional adjustment unit 116 in addition to the above-described components. The data additional adjustment unit 116 further downsamples the time-series data of an activity state output from the data adjustment unit 115, while assigning the priority to each state. In the fifth embodiment as well, a walking state is included in the activity state. The data of the activity state is a time series whose time is corrected by the time correction unit 107, and is formed by the data of the rising state and the lying state determined by the activity state determination unit 105 and corrected by the activity state correction unit 106 and the walking state specified by the walking period specifying unit 109. When the data of the activity state downsampled by the data adjustment unit 115 is further downsampled by the data additional adjustment unit 116, the number of data of the activity state can further be reduced by thinning.

The data additional adjustment unit 116 downsamples, at an interval of 30 min, the data of the activity state obtained by the data adjustment unit 115 at an interval of 1 min, thereby time-serially extracting 1 point in every 30 points. When executing the downsampling again in this way, if walking is included 10 points or more, the data additional adjustment unit 116 gives a priority to this, and this portion is determined as the walking state in the result of downsampling at an interval of 30 min. This is because if walking is done for about 1/3 of the period, leaving the walking as a history is intuitively appropriate for a life-log. If walking is not included, one of rising and lying with a higher occurrence frequency is given a priority.

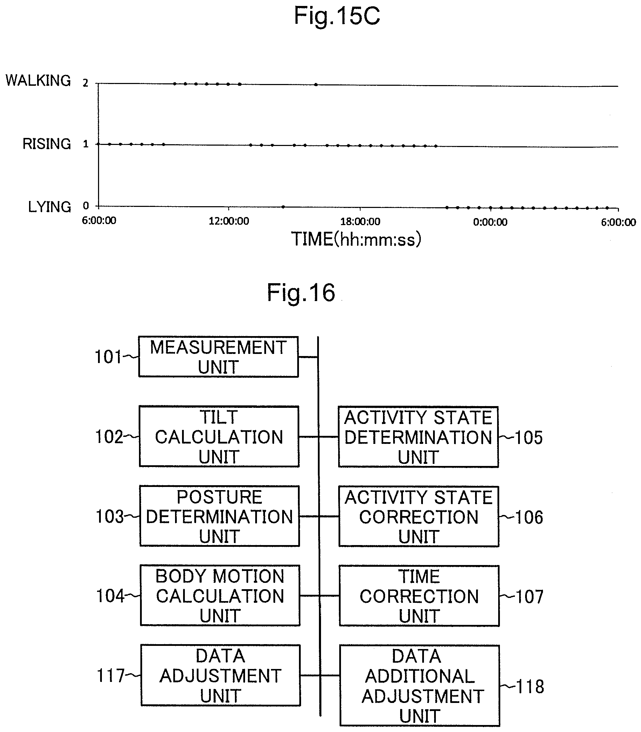

Effects according to this embodiment will be described with reference to Figs. 15A, 15B, and 15C. Fig. 15A shows the time-series data of an activity state before downsampling by the data adjustment unit 115 and the data additional adjustment unit 116. Fig. 15B shows the result of downsampling at an interval of 1 min by the data adjustment unit 115 and then downsampling at an interval of 30 min by the data additional adjustment unit 116. Fig. 15C shows the result of downsampling at an interval of 30 min by the data adjustment unit 115.

Referring to Figs. 15B and 15C, almost the same extraction results are obtained. In a case in which the data additional adjustment unit 116 performs downsampling at an interval of 30 min, since data downsampled (thinned) at an interval of 1 min is downsampled (thinned) at an interval of 30 min, a light operation suffices. To the contrary, in a case in which the data adjustment unit 115 performs downsampling at an interval of 30 min, data per sec needs to be stored for 30 min. This applies a load to arithmetic processing (the CPU or memory is oppressed) in a real-time operation. According to the fifth embodiment, it is possible to implement downsampling by an operation of little load.

[Sixth Embodiment]

The sixth embodiment of the present invention will be described next with reference to Fig. 16. An activity state analysis device includes a measurement unit 101, a tilt calculation unit 102, a posture determination unit 103, a body motion calculation unit 104, an activity state determination unit 105, an activity state correction unit 106, and a time correction unit 107. These components are the same as in the above-described first embodiment. The activity state analysis device according to the sixth embodiment includes a data adjustment unit 117 and a data additional adjustment unit 118 in addition to the above-described components.

The data adjustment unit 117 downsamples the time-serially obtained data of the activity state including a rising state (first state) and a lying state (second state), which are time-serially obtained, while assigning a priority to each state of the first state and the second state. The data adjustment unit 117 downsamples, for example, data per sec at an interval of 1 min. In this downsampling processing, the data adjustment unit 117 assigns a priority to each of rising and lying. If the rising and lying have the same point (time), the data adjustment unit 117 gives a priority to rising.

The data additional adjustment unit 118 further downsamples the time-series data of an activity state output from the data adjustment unit 117, while assigning the priority to each state.

The data of the activity state is a time series whose time is corrected by the time correction unit 107, and is formed by the rising state and the lying state determined by the activity state determination unit 105 and corrected by the activity state correction unit 106. When the data adjustment unit 117 and the data additional adjustment unit 118 perform downsampling, the number of data of the activity state can be reduced by thinning.

For example, in a situation in which a person can hardly walk immediately after an operation in a hospital, walking determination is unnecessary. In addition, since the body load is different between lying in which a person yields himself/herself to gravity and rising in which he/she rises against gravity, an application purpose focusing only on rising and lying can also be considered. In this case, as described above, the rising state and the lying state, which are time-serially obtained, are set to the target, and the number of data of the activity state is reduced by thinning.

As described above, according to the present invention, in addition to the magnitude of a body motion obtained by the body motion calculation unit, the activity state correction unit performs correction by providing a dead band, and the time correction unit corrects a delay caused by the correction. It is therefore possible to more correctly measure the activity state of the measurement target person with focus on the lifestyle.

Note that the present invention is not limited to the above-described embodiments, and many modification and combinations can be made by a person who has normal knowledge in the field without departing from the technical scope of the present invention. For example, the second embodiment and the third embodiment may be combined, as a matter of course. In addition, the first state may be a standing state, and the second state may be a sitting state. The scope of the present invention is limited by the scope of the appended claims.

Explanation of the Reference Numerals and Signs

101... measurement unit, 102...tilt calculation unit, 103...posture determination unit, 104...body motion calculation unit, 105... activity state determination unit, 106...activity state correction unit, 107...time correction unit

**Claims**

1. An activity state analysis device comprising:

   a measurement unit (101) configured to be attached to a measurement target person and configured to measure an acceleration;
   a tilt calculation unit (102) configured to obtain an angle of a tilt of an upper part of a body of the measurement target person based on the acceleration measured by the measurement unit (101);
   a posture determination unit (103) configured to determine a posture of the measurement target person based on the angle of the tilt obtained by the tilt calculation unit (102);
   a body motion calculation unit (104) configured to obtain a magnitude of a body motion of the measurement target person based on the acceleration measured by the measurement unit (101);
   **characterized by**
   an activity state determination unit (105) configured to determine, based on the posture determined by the posture determination unit (103) and the magnitude of the body motion calculated by the body motion calculation unit (104), whether an activity state of the measurement target person is a first state or a second state different from the first state;
   an activity state correction unit (106) configured to, if, in a time series of the activity state obtained by the activity state determination unit (105), the activity state transitions from one state of the first state and the second state to the other state, and the other state continues for a predetermined time defined in advance, determine that the activity state has transitioned from the one state to the other state, and if the other state does not continue for the predetermined time, determine that the activity state has not transitioned from the one state to the other state; and
   a time correction unit (107) configured to, when the activity state correction unit (106) determines that the activity state has transitioned from the one state to the other state, set a time set back by the predetermined time from a time at which the activity state correction unit (106) determines that the activity state has transitioned from the one state to the other state, as a transition time at which the activity state has transitioned from the one state to the other state.

2. The activity state analysis device according to claim 1, wherein the measurement unit (101) is configured to measure accelerations in three directions along X-, Y-, and Z-axes that are orthogonal to each other.

3. The activity state analysis device according to claim 1 or 2, further comprising:

   a walking pace calculation unit (108) configured to obtain a walking pace of the measurement target person based on the acceleration measured by the measurement unit (101); and
   a walking period specifying unit (109) configured to specify, based on the walking pace of the measurement target person obtained by the walking pace calculation unit (108), a period of a walking state in which the measurement target person was walking in a period of the first state defined by the transition time corrected by the time correction unit (107).

4. The activity state analysis device according to any one of claims 1 to 3, further comprising a data adjustment unit (115) configured to downsample data to reduce the number of data of the activity state from per second to per 1 minute and to assign a priority to each of walking, rising and lying giving a high priority to walking, wherein the data adjustment unit (115) is configured to

   hold 1 minute of data of the activity state obtained at an interval of 1 second ,
   determine the data of the activity state of one point after downsampling as walking, if walking is determined continuously for 6 seconds in the period of 1 minute of holding,
   employ one of rising and lying whichever is included longer in the period of 1 minute of holding as the data of one point after downsampling, if walking is not determined continuously for 6 seconds, and
   give a priority to rising, if rising and lying have a same time.

5. The activity state analysis device according to claim 4, further comprising a data additional adjustment unit (116) configured to downsample, at an interval of 30 minutes, the data of the activity state downsampled by the data adjustment unit (115), wherein the data additional adjustment unit (116) configured to
   determine a status in a 30-minute period

by giving a priority to walking such that the status is determined as walking, if walking is included 10 points or more in the period, and

by giving a priority to one of rising and lying whichever has a higher occurrence frequency, if walking is not included in the period.

6. The activity state analysis device according to any one of claims 1 to 5, further comprising:

a physical information measurement unit configured to measure physical information of the measurement target person; and

a statistic value calculation unit (113) configured to obtain, for each activity state defined by the transition time corrected by the time correction unit (107), a statistic value including at least one of an average value, a median, a maximum value, a minimum value, a standard deviation, a 75% level value, and a 25% level value of the physical information measured by the physical information measurement unit.

7. The activity state analysis device according to any one of claims 1 to 5, further comprising:

a heart rate measurement unit configured to measure a heart rate of the measurement target person;

a motion intensity calculation unit (114) configured to calculate a motion intensity of the measurement target person based on the heart rate calculated by the heart rate measurement unit; and

a statistic value calculation unit (113a) configured to obtain, for each activity state defined by the transition time corrected by the time correction unit (107), a statistic value including at least one of an average value, a median, a maximum value, a minimum value, a standard deviation, a 75% level value, and a 25% level value of the motion intensity obtained by the motion intensity calculation unit (114).

8. The activity state analysis device according to any one of claims 1 to 7, wherin the activity state determination unit (105) is configured to determine whether the activity state of the measurement target person is a rising state in which the measurement target person gets up as the first state or a lying state in which the measurement target person is lying in a bed as the second state.

9. An activity state analysis method comprising:

a first step of measuring an acceleration in an action of a measurement target person;

a second step of obtaining an angle of a tilt of an upper part of a body of the measurement target person based on the acceleration measured in the first step;

a third step of determining a posture of the measurement target person based on the angle of the tilt obtained in the second step;

a fourth step of obtaining a magnitude of a body motion of the measurement target person based on the acceleration measured in the first step;

**characterized by**

a fifth step of determining, based on the posture determined in the third step and the magnitude of the body motion calculated in the fourth step, whether an activity state of the measurement target person is a first state or a second state different from the first state;

a sixth step of, if, in a time series of the activity state obtained in the fifth step, the activity state transitions from one state of the first state and the second state to the other state, and the other state continues for a predetermined time defined in advance, determining that the activity state has transitioned from the one state to the other state, and if the other state does not continue for the predetermined time, determining that the activity state has not transitioned from the one state to the other state; and

a seventh step of, when it is determined in the sixth step that the activity state has transitioned from the one state to the other state, setting a time set back by the predetermined time from a time at which it is determined in the sixth step that the activity state has transitioned from the one state to the other state, as a transition time at which the activity state has transitioned from the one state to the other state.

10. The activity state analysis method according to claim 9, wherein, in the first step, accelerations in three directions along X-, Y-, and Z-axes that are orthogonal to each other are measured.

11. The activity state analysis method according to claim 9 or 10, further comprising:

an eighth step of obtaining a walking pace of the measurement target person based on the acceleration measured in the first step; and

a ninth step of specifying, based on the walking pace of the measurement target person obtained in the eighth step, a period of a walking state in which the measurement target person was walking in a period of the first state defined by the transition time corrected in the seventh step.

12. The activity state analysis method according to any one of claims 9 to 11, further comprising:

a 10th step of measuring physical information of the measurement target person; and
an 11th step of obtaining, for each the activity state defined by the transition time corrected in the seventh step, a statistic value including at least one of an average value, a median, a maximum value, a minimum value, a standard deviation, a 75% level value, and a 25% level value of the physical information measured in the 10th step.

13. The activity state analysis method according to any one of claims 9 to 11, further comprising:

a 10th step of measuring a heart rate of the measurement target person;
an 11th step of calculating a motion intensity of the measurement target person based on the heart rate calculated in the 10th step; and
a 13th step of obtaining, for each the activity state defined by the transition time corrected in the seventh step, a statistic value including at least one of an average value, a median, a maximum value, a minimum value, a standard deviation, a 75% level value, and a 25% level value of the motion intensity obtained in the 11th step.

14. The activity state analysis method according to any one of claims 9 to 13, wherein the first state is a rising state in which the measurement target person gets up, and the second state is a lying state in which the measurement target person is lying in a bed.


**Patentansprüche**

1. Vorrichtung zur Analyse des Aktivitätszustands, umfassend:

eine Messeinheit (101), die so konfiguriert ist, dass sie an einer zu Messzielperson angebracht werden kann und so konfiguriert ist, dass sie eine Beschleunigung misst;
eine Neigungsberechnungseinheit (102), die so konfiguriert ist, dass sie auf der Grundlage der von der Messeinheit (101) gemessenen Beschleunigung einen Neigungswinkel eines oberen Teils eines Körpers der Messzielperson erhält;
eine Körperhaltungsbestimmungseinheit (103), die so konfiguriert ist, dass sie auf der Grundlage des von der Neigungsberechnungseinheit (102) erhaltenen Neigungswinkels eine Körperhaltung der Messzielperson bestimmt;
eine Körperbewegungsberechnungseinheit (104), die so konfiguriert ist, dass sie auf der Grundlage der von der Messeinheit (101) gemessenen Beschleunigung ein Ausmaß einer Körperbewegung der Messzielperson ermittelt;
**gekennzeichnet durch**
eine Aktivitätszustandsbestimmungseinheit (105), die so konfiguriert ist, dass sie auf der Grundlage der von der Körperhaltungsbestimmungseinheit (103) bestimmten Körperhaltung und dem Ausmaß der von der Körperbewegungsberechnungseinheit (104) berechneten Körperbewegung bestimmt, ob ein Aktivitätszustand der Messzielperson ein erster Zustand oder ein zweiter, sich vom ersten Zustand unterscheidender Zustand ist;
eine Aktivitätszustandskorrektureinheit (106), die so konfiguriert ist, dass sie bestimmt, dass der Aktivitätszustand vom einen Zustand in den jeweils anderen Zustand übergegangen ist, wenn in einer Zeitreihe des von der Aktivitätszustandsbestimmungseinheit (105) erhaltenen Aktivitätszustands der Aktivitätszustand von einem Zustand eines des ersten Zustands und des zweiten Zustands in den jeweils anderen Zustand übergeht und der jeweils andere Zustand für eine im Voraus festgelegte Zeit andauert, und bestimmt, dass der Aktivitätszustand nicht vom einen Zustand in den jeweils anderen Zustand übergegangen ist, wenn der jeweils andere Zustand nicht für die im Voraus festgelegte Zeit andauert; und
eine Zeitkorrektureinheit (107), die so konfiguriert ist, dass sie, wenn die Aktivitätszustandskorrektureinheit (106) bestimmt, dass der Aktivitätszustand vom einen Zustand in den jeweils anderen Zustand übergegangen ist, eine Zeit, die um die vorbestimmte Zeit von einem Zeitpunkt zurückgesetzt ist, zu dem die Aktivitätszustandskorrektureinheit (106) bestimmt, dass der Aktivitätszustand vom einen Zustand in den jeweils anderen Zustand übergegangen ist, als eine Übergangszeit festlegt, zu der der Aktivitätszustand vom einen Zustand in den jeweils anderen Zustand übergegangen ist.

2. Vorrichtung zur Analyse des Aktivitätszustands nach Anspruch 1, wobei die Messeinheit (101) so konfiguriert ist, dass sie Beschleunigungen in drei Richtungen entlang X-, Y- und Z-Achsen misst, die orthogonal zueinander sind.

3. Vorrichtung zur Analyse des Aktivitätszustands nach Anspruch 1 oder 2, die ferner umfasst:

   eine Gehgeschwindigkeitsberechnungseinheit (108), die so konfiguriert ist, dass sie auf der Grundlage der von der Messeinheit (101) gemessenen Beschleunigung eine Gehgeschwindigkeit der Messzielperson ermittelt; und
   eine Gehzeitspannenspezifizierungseinheit (109), die so konfiguriert ist, dass sie auf der Grundlage der von der Gehgeschwindigkeitsberechnungseinheit (108) erhaltenen Gehgeschwindigkeit der Messzielperson eine Zeitspanne eines Gehzustands spezifiziert, in der die Messzielperson in einer durch die von der Zeitkorrektureinheit (107) korrigierte Übergangszeit definierten Zeitspanne des ersten Zustands gegangen ist.

4. Vorrichtung zur Analyse des Aktivitätszustands nach einem der Ansprüche 1 bis 3, die ferner eine Datenanpassungseinheit (115) umfasst, die so konfiguriert ist, dass sie die pro Sekunde erfassten Daten des Aktivitätszustands in einem Intervall von 1 Minute heruntersampelt, wobei die Datenanpassungseinheit (115) so konfiguriert ist, dass sie

   die Daten pro Sekunde für 1 Minute hält,
   Daten eines Punktes nach dem Heruntersampeln als Gehen bestimmt, wenn Gehen kontinuierlich für 6 Sekunden innerhalb von 1 Minute des Haltens bestimmt wird,
   ein längeres von in 1 Minute enthaltenem Aufstehen und Liegen als die Daten eines Punktes nach dem Heruntersampeln verwendet, wenn nicht kontinuierlich für 6 Sekunden Gehen bestimmt wird, und
   dem Aufstehen den Vorrang geben, wenn Aufstehen und Liegen zeitlich übereinstimmen.

5. Vorrichtung zur Analyse des Aktivitätszustands nach Anspruch 4, die ferner eine zusätzliche Datenanpassungseinheit (116) umfasst, die so konfiguriert ist, dass sie in einem Intervall von 30 Minuten die Daten des Aktivitätszustands, die von der Datenanpassungseinheit (115) heruntergesampelt wurden, heruntersampelt, wobei die zusätzliche Datenanpassungseinheit (116) so konfiguriert ist, dass sie

   einen Status in einer 30-minütigen Zeitspanne als Gehen bestimmt, wenn in der Zeitspanne Gehen mit 10 oder mehr Punkten enthalten ist, und
   eine Priorität einem von Aufstehen und Liegen mit einer höheren Häufigkeit des Auftretens gibt, wenn in der Zeitspanne Gehen nicht enthalten ist.

6. Vorrichtung zur Analyse des Aktivitätszustands nach einem der Ansprüche 1 bis 5, die ferner umfasst:

   eine Messeinheit für physikalische Informationen, die so konfiguriert ist, dass sie physikalische Informationen der Messzielperson misst; und
   eine Statistikwertberechnungseinheit (113), die so konfiguriert ist, dass sie für jeden durch die von der Zeitkorrektureinheit (107) korrigierte Übergangszeit definierten Aktivitätszustand einen Statistikwert ermittelt, der mindestens einen Durchschnittswert, einen Median, einen Maximalwert, einen Minimalwert, eine Standardabweichung, einen 75%-Niveauwert und einen 25%-Niveauwert der von der Messeinheit für physikalische Information gemessenen physikalischen Information enthält.

7. Vorrichtung zur Analyse des Aktivitätszustands nach einem der Ansprüche 1 bis 5, die ferner umfasst:

   eine Herzfrequenzmesseinheit, die so konfiguriert ist, dass sie eine Herzfrequenz der Messzielperson misst;
   eine Bewegungsintensitätsberechnungseinheit (114), die so konfiguriert ist, dass sie auf der Grundlage der von der Herzfrequenzmesseinheit berechneten Herzfrequenz eine Bewegungsintensität der Messzielperson berechnet; und
   eine Statistikwertberechnungseinheit (113a), die so konfiguriert ist, dass sie für jeden durch die von der Zeitkorrektureinheit (107) korrigierte Übergangszeit definierten Bewegungszustand, einen Statistikwert ermittelt, der mindestens einen Durchschnittswert, einen Median, einen Maximalwert, einen Minimalwert, eine Standardabweichung, einen 75%-Niveauwert und einen 25%-Niveauwert der von der Bewegungsintensitätsberechnungseinheit (114) ermittelten Bewegungsintensität enthält.

**8.** Vorrichtung zur Analyse des Aktivitätszustands nach einem der Ansprüche 1 bis 7, wobei die Aktivitätszustandsbestimmungseinheit (105) so konfiguriert ist, dass sie bestimmt, ob der Aktivitätszustand der Messzielperson als erster Zustand ein Aufstehzustand ist, in dem die Messzielperson aufsteht, oder als zweiter Zustand ein Liegezustand ist, in dem die Messzielperson in einem Bett liegt.

**9.** Ein Verfahren zur Analyse des Aktivitätszustandes, das Folgendes umfasst:

einen ersten Schritt des Messens einer Beschleunigung bei einer Aktion einer Messzielperson;
einen zweiten Schritt des auf der im ersten Schritt gemessenen Beschleunigung basierenden Ermittelns eines Neigungswinkels eines oberen Teils eines Körpers der Messzielperson;
einen dritten Schritt des auf dem im zweiten Schritt erhaltenen Neigungswinkel basierenden Bestimmens einer Körperhaltung der Messzielperson;
einen vierten Schritt des auf der im ersten Schritt gemessenen Beschleunigung basierenden Ermittelns eines Ausmaßes einer Körperbewegung der Messzielperson;
**gekennzeichnet durch**
einen fünften Schritt des auf der im dritten Schritt bestimmten Körperhaltung und dem im vierten Schritt berechneten Ausmaß der Körperbewegung basierenden Bestimmens, ob ein Aktivitätszustand der Messzielperson ein erster Zustand oder ein zweiter, sich vom ersten Zustand unterscheidender Zustand ist;
einen sechsten Schritt des Bestimmens, dass der Aktivitätszustand vom einen Zustand in den jeweils anderen Zustand übergegangen ist, wenn in einer Zeitreihe des im fünften Schritt erhaltenen Aktivitätszustands der Aktivitätszustand von einem Zustand eines des ersten Zustands und des zweiten Zustands in den jeweils anderen Zustand übergeht und der jeweils andere Zustand für eine im Voraus festgelegte Zeit andauert, und des Bestimmens, dass der Aktivitätszustand nicht vom einen Zustand in den jeweils anderen Zustand übergegangen ist, wenn der jeweils andere Zustand nicht für die im Voraus festgelegte Zeit andauert; und
einen siebten Schritt, bei dem, wenn im sechsten Schritt bestimmt wird, dass der Aktivitätszustand vom einen Zustand in den jeweils anderen Zustand übergegangen ist, eine Zeit, die um die vorbestimmte Zeit von einem Zeitpunkt zurückgesetzt ist, zu dem im sechsten Schritt bestimmt wird, dass der Aktivitätszustand von dem einen Zustand in den jeweils anderen Zustand übergegangen ist, als eine Übergangszeit festgelegt wird, zu der der Aktivitätszustand vom einen Zustand in den anderen Zustand übergegangen ist.

**10.** Verfahren zur Analyse des Aktivitätszustands nach Anspruch 9, wobei im ersten Schritt Beschleunigungen in drei Richtungen entlang von X-, Y- und Z-Achsen gemessen werden, die orthogonal zueinander sind.

**11.** Verfahren zur Analyse des Aktivitätszustands nach Anspruch 9 oder 10, das ferner umfasst:

einen achten Schritt des Ermittelns eines Gehtempos der Messzielperson basierend auf der im ersten Schritt gemessenen Beschleunigung; und
einen neunten Schritt des auf der im achten Schritt ermittelten Gehgeschwindigkeit der Messzielperson basierenden Spezifizierens einer Zeitspanne eines Gehzustands, in dem die Messzielperson während einer durch die im siebten Schritt korrigierte Übergangszeit definierten Zeitspanne des ersten Zustands gegangen ist.

**12.** Verfahren zur Analyse des Aktivitätszustands nach einem der Ansprüche 9 bis 11, ferner umfassend:

einen 10. Schritt des Messens physischer Informationen der Messzielperson; und
einen 11. Schritt, in dem für jeden durch die im siebten Schritt korrigierte Übergangszeit definierten Aktivitätszustand ein Statistikwert ermittelt wird, der mindestens einen Durchschnittswert, einen Median, einen Maximalwert, einen Minimalwert, eine Standardabweichung, einen 75 %-Niveauwert und einen 25 %-Niveauwert der im zehnten Schritt gemessenen physischen Information enthält.

**13.** Verfahren zur Analyse des Aktivitätszustands nach einem der Ansprüche 9 bis 11, das ferner umfasst:

einen 10. Schritt des Messens einer Herzfrequenz der Messzielperson;
einen 11. Schritt zum Berechnen einer Bewegungsintensität der Messzielperson auf der Grundlage der im 10. Schritt berechneten Herzfrequenz; und
einen 13. Schritt, in dem für jeden Aktivitätszustand, der durch die im siebten Schritt korrigierte Übergangszeit definiert ist, ein Statistikwert ermittelt wird, der mindestens einen Durchschnittswert, einen Median, einen Maximalwert, einen Minimalwert, eine Standardabweichung, einen 75%-Niveau-Wert und einen 25%-Niveau-Wert der im 11. Schritt ermittelten Bewegungsintensität enthält.

**14.** Verfahren zur Aktivitätszustandsanalyse nach einem der Ansprüche 9 bis 13, wobei der erste Zustand ein Aufstehzustand ist, in dem die Messzielperson aufsteht, und der zweite Zustand ein Liegezustand ist, in dem die Messzielperson in einem Bett liegt.

**Revendications**

**1.** Dispositif d'analyse d'état d'activité comprenant :

une unité de mesure (101) configurée pour être fixée à une personne cible de mesure et configurée pour mesurer une accélération ;
une unité de calcul d'inclinaison (102) configurée pour obtenir un angle d'une inclinaison d'une partie supérieure d'un corps de la personne cible de mesure sur la base de l'accélération mesurée par l'unité de mesure (101) ;
une unité de détermination de posture (103) configurée pour déterminer une posture de la personne cible de mesure sur la base de l'angle de l'inclinaison obtenu par l'unité de calcul d'inclinaison (102) ;
une unité de calcul de mouvement corporel (104) configurée pour obtenir une amplitude d'un mouvement corporel de la personne cible de mesure sur la base de l'accélération mesurée par l'unité de mesure (101) ;
**caractérisé par**
une unité de détermination d'état d'activité (105) configurée pour déterminer, sur la base de la posture déterminée par l'unité de détermination de posture (103) et l'amplitude du mouvement corporel calculée par l'unité de calcul de mouvement corporel (104), si un état d'activité de la personne cible de mesure est un premier état ou un deuxième état différent du premier état ;
une unité de correction d'état d'activité (106) configurée pour, si, dans une série temporelle de l'état d'activité obtenu par l'unité de détermination d'état d'activité (105), l'état d'activité passe d'un état du premier état et du deuxième état à l'autre état, et l'autre état continue pendant une durée prédéterminée définie à l'avance, déterminer que l'état d'activité est passé de l'un état à l'autre état, et si l'autre état ne continue pas pendant la durée prédéterminée, déterminer que l'état d'activité n'est pas passé de l'un état à l'autre état ; et
une unité de correction de durée (107) configurée pour, lorsque l'unité de correction d'état d'activité (106) détermine que l'état d'activité est passé de l'un état à l'autre état, régler un retard temporel par la durée prédéterminée à partir d'une durée à laquelle l'unité de correction d'état d'activité (106) détermine que l'état d'activité est passé de l'un état à l'autre état, comme une durée de transition à laquelle l'état d'activité est passé de l'un état à l'autre état.

**2.** Dispositif d'analyse d'état d'activité selon la revendication 1, dans lequel l'unité de mesure (101) est configurée pour mesurer les accélérations dans trois directions le long d'axes X, Y et Z qui sont perpendiculaires les uns aux autres.

**3.** Dispositif d'analyse d'état d'activité selon la revendication 1 ou 2, comprenant en outre :

une unité de calcul de rythme de marche (108) configurée pour obtenir un rythme de marche de la personne cible de mesure sur la base de l'accélération mesurée par l'unité de mesure (101) ; et
une unité de spécification de durée de marche (109) configurée pour spécifier, sur la base du rythme de marche de la personne cible de mesure obtenu par l'unité de calcul de rythme de marche (108), une durée d'un état de marche dans laquelle la personne cible de mesure marchait dans une période du premier état défini par la durée de transition corrigée par l'unité de correction de durée (107).

**4.** Dispositif d'analyse d'état d'activité selon l'une quelconque des revendications 1 à 3, comprenant en outre une unité de réglage de données (115) configurée pour sous-échantillonner des données pour réduire le nombre de données de l'état d'activité allant de données par seconde à donnés par minute et pour assigner une priorité à chacun de la marche, du lever et du coucher en donnant une priorité élevée à la marche, dans lequel l'unité de réglage de données (115) est configurée pour :

maintenir une minute de données de l'état d'activité obtenu à un intervalle d'une seconde,
déterminer les données de l'état d'activité d'un point après le sous-échantillonnage comme la marche, si la marche est déterminée de manière continue pendant six secondes dans la période d'une minute de maintien,
utiliser l'un du lever et du coucher selon celui qui est inclus le plus longtemps dans la période d'une minute de maintien comme les données d'un point après le sous-échantillonnage, si la marche n'est pas déterminée de manière continue pendant six secondes, et
donner une priorité au lever, si le lever et le coucher présentent une même durée.

**5.** Dispositif d'analyse d'état d'activité selon la revendication 4, comprenant en outre une unité de réglage additionnel de données (116) configurée pour sous-échantillonner, à un intervalle de trente minutes, les données de l'état d'activité sous-échantillonné par l'unité de réglage de données (115), dans lequel l'unité de réglage additionnel de données (116) est configurée pour :

déterminer un statut dans une période de trente minutes en donnant une priorité à la marche de telle sorte que le statut est déterminé comme la marche si la marche est déterminée à dix points ou plus dans la période, et en donnant une priorité à l'un du lever et du coucher selon celui qui présente une fréquence d'occurrence plus élevée, si la marche n'est pas incluse dans la période.

**6.** Dispositif d'analyse d'état d'activité selon l'une quelconque des revendications 1 à 5, comprenant en outre :

une unité de mesure d'informations physiques configurée pour mesurer des informations physiques de la personne cible de mesure ; et
une unité de calcul de valeur statistique (113) configurée pour obtenir, pour chaque état d'activité défini par la durée de transition corrigée par l'unité de correction de durée (107), une valeur statistique comportant au moins l'une d'une valeur moyenne, d'une valeur médiane, d'une valeur maximale, d'une valeur minimale, d'un écart type, d'une valeur de niveau de 75 %, et d'une valeur de niveau de 25 % des informations physiques mesurées par l'unité de mesure d'informations physiques.

**7.** Dispositif d'analyse d'état d'activité selon l'une quelconque des revendications 1 à 5, comprenant en outre :

une unité de mesure de fréquence cardiaque configurée pour mesurer une fréquence cardiaque de la personne cible de mesure ;
une unité de calcul d'intensité de mouvement (114) configurée pour calculer une intensité de mouvement de la personne cible de mesure sur la base de la fréquence cardiaque calculée par l'unité de mesure de fréquence cardiaque ; et
une unité de calcul de valeur statistique (113a) configurée pour obtenir, pour chaque état d'activité défini par la durée de transition corrigée par l'unité de correction de durée (107), une valeur statistique comportant au moins l'une d'une valeur moyenne, d'une valeur médiane, d'une valeur maximale, d'une valeur minimale, d'un écart type, d'une valeur de niveau de 75 %, et d'une valeur de niveau de 25 % de l'intensité de mouvement obtenue par l'unité de calcul d'intensité de mouvement (114).

**8.** Dispositif d'analyse d'état d'activité selon l'une quelconque des revendications 1 à 7, dans lequel l'unité de détermination d'état d'activité (105) est configurée pour déterminer si l'état d'activité de la personne cible de mesure est un état de lever dans lequel la personne cible de mesure se lève comme le premier état, ou un état de coucher dans lequel la personne cible de mesure est couchée dans un lit comme le deuxième état.

**9.** Procédé d'analyse d'état d'activité comprenant :

une première étape de mesure d'une accélération dans une action d'une personne cible de mesure ;
une deuxième étape d'obtention d'un angle d'une inclinaison d'une partie supérieure d'un corps de la personne cible de mesure sur la base de l'accélération mesurée dans la première étape ;
une troisième étape de détermination d'une posture de la personne cible de mesure sur la base de l'angle de l'inclinaison obtenu dans la deuxième étape ;
une quatrième étape d'obtention d'une amplitude d'un mouvement corporel de la personne cible de mesure sur la base de l'accélération mesurée dans la première étape ;
**caractérisé par**
une cinquième étape de détermination, sur la base de la posture déterminée dans la troisième étape et de l'amplitude du mouvement corporel calculé dans la quatrième étape, si un état d'activité de la personne cible de mesure est un premier état ou un deuxième état différent du premier état ;
une sixième étape de, si, dans une série temporelle de l'état d'activité obtenu dans la cinquième étape, l'état d'activité passe d'un état du premier état et du deuxième état à l'autre état, et l'autre état continue pendant une durée prédéterminée défini à l'avance, détermination que l'état d'activité est passé de l'un état à l'autre état, et si l'autre état ne continue pas pendant la durée prédéterminée, détermination que l'état d'activité n'est pas passé de l'un état à l'autre état ; et
une septième étape de, lorsqu'il est déterminé dans la sixième étape que l'état d'activité est passé de l'un état à l'autre état, réglage d'un retard temporel par la durée prédéterminée à partir d'une durée à laquelle il est déterminé dans la sixième étape que l'état d'activité est passé de l'un état à l'autre état, comme une durée de transition à laquelle l'état d'activité est passé de l'un état à l'autre état.

10. Procédé d'analyse d'état d'activité selon la revendication 9, dans lequel, dans la première étape, les accélérations dans trois directions le long d'axes X, Y et Z qui sont perpendiculaires les uns aux autres sont mesurées.

11. Procédé d'analyse d'état d'activité selon la revendication 9 ou 10, comprenant en outre :

une huitième étape d'obtention d'un rythme de marche de la personne cible de mesure sur la base de l'accélération mesurée dans la première étape ; et
une neuvième étape de spécification, sur la base du rythme de marche de la personne cible de mesure obtenu dans la huitième étape, d'une période d'un état de marche dans laquelle la personne cible de mesure marchait dans une période du premier état défini par la durée de transition corrigée dans la septième étape.

12. Procédé d'analyse d'état d'activité selon l'une quelconque des revendications 9 à 11, comprenant en outre :

une 10e étape de mesure d'informations physiques de la personne cible de mesure ; et
une 11e étape d'obtention, pour chacun de l'état d'activité défini par la durée de transition corrigée dans la septième étape, d'une valeur statistique comportant au moins l'une d'une valeur moyenne, d'une valeur médiane, d'une valeur maximale, d'une valeur minimale, d'un écart type, d'une valeur de niveau de 75 %, et d'une valeur de niveau de 25 % des informations physiques mesurées dans la 10e étape.

13. Procédé d'analyse d'état d'activité selon l'une quelconque des revendications 9 à 11, comprenant en outre :

une 10e étape de mesure d'une fréquence cardiaque de la personne cible de mesure ;
une 11e étape de calcul d'une intensité de mouvement de la personne cible de mesure sur la base de la fréquence cardiaque calculée dans la 10e étape ; et
une 13e étape d'obtention, pour chacun de l'état d'activité défini par la durée de transition corrigée dans la septième étape, d'une valeur statistique comportant au moins l'une d'une valeur moyenne, d'une valeur médiane, d'une valeur maximale, d'une valeur minimale, d'un écart type, d'une valeur de niveau de 75 %, et d'une valeur de niveau de 25 % de l'intensité de mouvement obtenue dans la 11e étape.

14. Procédé d'analyse d'état d'activité selon l'une quelconque des revendications 9 à 13, dans lequel le premier état est un état de lever dans lequel la personne cible de mesure se lève, et le deuxième état est un état de coucher dans lequel la personne cible de mesure est couchée dans un lit.

## Fig.1

| | |
|---|---|
| 101~ MEASUREMENT UNIT | |
| 102~ TILT CALCULATION UNIT | ACTIVITY STATE DETERMINATION UNIT ~105 |
| 103~ POSTURE DETERMINATION UNIT | ACTIVITY STATE CORRECTION UNIT ~106 |
| 104~ BODY MOTION CALCULATION UNIT | TIME CORRECTION UNIT ~107 |

## Fig.2A

RISING 1 / 0 / LYING -1

ELAPSED TIME [SEC]

## Fig.2B

DETECTION ERROR

fi

RISING 1 / 0 / LYING -1

ELAPSED TIME [SEC]

## Fig.2C

gi

RISING 1 / 0 / LYING -1

ELAPSED TIME [SEC]

## Fig.2D

g'i

RISING 1 / 0 / LYING -1

ELAPSED TIME [SEC]

# Fig.3

LYING FACE DOWN→TEMPORARY POSTURE CHANGE

POSTURE

LYING DURING SLEEP
CANNOT BE READ
AT A GLANCE

# Fig.4

# Fig.5

## Fig.6

**SENSOR TERMINAL** 202

ACCELERATION SENSOR 301

DETECTION UNIT 302

STORAGE UNIT 303

ANALYSIS UNIT 304

TRANSMISSION PROCESSING UNIT 305

COMMUNICATION INTERFACE 306

**RELAY TERMINAL** 203

COMMUNICATION INTERFACE 311

RECEPTION PROCESSING UNIT 312

STORAGE UNIT 313

ANALYSIS UNIT 314

TRANSMISSION PROCESSING UNIT 315

COMMUNICATION INTERFACE 316

COMMUNICATION INTERFACE 321

RECEPTION PROCESSING UNIT 322

STORAGE UNIT 323

ANALYSIS UNIT 324

CONTROL UNIT 325

OPERATION DEVICE 326

EXTERNAL TERMINAL 204

# Fig.7

```
101 — MEASUREMENT
        UNIT

102 — TILT                  ACTIVITY STATE
        CALCULATION          DETERMINATION — 105
        UNIT                 UNIT

103 — POSTURE               ACTIVITY STATE
        DETERMINATION        CORRECTION — 106
        UNIT                 UNIT

104 — BODY MOTION           TIME
        CALCULATION          CORRECTION — 107
        UNIT                 UNIT

108 — WALKING PACE          WALKING PERIOD
        CALCULATION          SPECIFYING — 109
        UNIT                 UNIT
```

# Fig.8

## Fig.9

| | | |
|---|---|---|
| 101~ | MEASUREMENT UNIT | ELECTRO-CARDIOGRAPHIC UNIT ~111 |
| 102~ | TILT CALCULATION UNIT | ACTIVITY STATE DETERMINATION UNIT ~105 |
| 103~ | POSTURE DETERMINATION UNIT | ACTIVITY STATE CORRECTION UNIT ~106 |
| 104~ | BODY MOTION CALCULATION UNIT | TIME CORRECTION UNIT ~107 |
| 112~ | HEARTBEAT CALCULATION UNIT | STATISTIC VALUE CALCULATION UNIT ~113 |

## Fig.10

HEARTBEAT RATE (bpm) vs NUMBER OF DAYS OF MEASUREMENT (DAY)

# Fig.11

| | |
|---|---|
| 101~ MEASUREMENT UNIT | ELECTRO-CARDIOGRAPHIC UNIT ~111 |
| 102~ TILT CALCULATION UNIT | ACTIVITY STATE DETERMINATION UNIT ~105 |
| 103~ POSTURE DETERMINATION UNIT | ACTIVITY STATE CORRECTION UNIT ~106 |
| 104~ BODY MOTION CALCULATION UNIT | TIME CORRECTION UNIT ~107 |
| 112~ HEARTBEAT CALCULATION UNIT | MOTION INTENSITY CALCULATION UNIT ~114 |
| | STATISTIC VALUE CALCULATION UNIT ~113a |

Fig.12

| | |
|---|---|
| 101~ MEASUREMENT UNIT | |
| 102~ TILT CALCULATION UNIT | ACTIVITY STATE DETERMINATION UNIT ~105 |
| 103~ POSTURE DETERMINATION UNIT | ACTIVITY STATE CORRECTION UNIT ~106 |
| 104~ BODY MOTION CALCULATION UNIT | TIME CORRECTION UNIT ~107 |
| 108~ WALKING PACE CALCULATION UNIT | WALKING PERIOD SPECIFYING UNIT ~109 |
| 115~ DATA ADJUSTMENT UNIT | |

## Fig.13A

## Fig.13B

## Fig.13C

## Fig.14

```
101~┌─────────────┐
     │ MEASUREMENT │
     │    UNIT     │
     └─────────────┘

102~┌─────────────┐         ┌─────────────────┐
     │    TILT     │         │ ACTIVITY STATE  │
     │ CALCULATION │         │  DETERMINATION  │~105
     │    UNIT     │         │      UNIT       │
     └─────────────┘         └─────────────────┘

103~┌─────────────┐         ┌─────────────────┐
     │   POSTURE   │         │ ACTIVITY STATE  │
     │DETERMINATION│         │   CORRECTION    │~106
     │    UNIT     │         │      UNIT       │
     └─────────────┘         └─────────────────┘

104~┌─────────────┐         ┌─────────────────┐
     │ BODY MOTION │         │      TIME       │
     │ CALCULATION │         │   CORRECTION    │~107
     │    UNIT     │         │      UNIT       │
     └─────────────┘         └─────────────────┘

108~┌─────────────┐         ┌─────────────────┐
     │WALKING PACE │         │ WALKING PERIOD  │
     │ CALCULATION │         │   SPECIFYING    │~109
     │    UNIT     │         │      UNIT       │
     └─────────────┘         └─────────────────┘

115~┌─────────────┐         ┌─────────────────┐
     │    DATA     │         │      DATA       │
     │ ADJUSTMENT  │         │   ADDITIONAL    │~116
     │    UNIT     │         │   ADJUSTMENT    │
     └─────────────┘         │      UNIT       │
                             └─────────────────┘
```

## Fig.15A

## Fig.15B

Fig.15C

Fig.16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015126822 A1
- US 2012274554 A1
- US 2016100776 A1
- US 2014313030 A1
- JP 2016182160 A

**Non-patent literature cited in the description**

- hitoe transmitter SDK API manual. NTT DOCOMO, Inc, 14 November 2016

- **KEN KODA.** Meanings and Importance of Active Bed Leaving and Exercise Load and Its Physiological Mechanism. *Human Resources Development Study Group for Community Rehabilitation,* 2013, http://wakayama-med-reha.com/wp-content/uploads/2013/12/31bc2df5f92f959aaabc9d676a556abe .pdf